(19) Europäisches Patentamt
European Patent Office
Office européen des brevets

(11) Publication number: **0 610 943 A1**

(12) **EUROPEAN PATENT APPLICATION**

(21) Application number: **94102138.8**

(22) Date of filing: **11.02.94**

(51) Int. Cl.5: **C07D 213/82**, A61K 31/44, C07D 413/12, C07D 401/12, //(C07D401/12,213:00,215:00), (C07D413/12,213:00,263:00)

Amended claims in accordance with Rule 86(2) EPC

(30) Priority: **11.02.93 ES 9300269**

(43) Date of publication of application: **17.08.94 Bulletin 94/33**

(84) Designated Contracting States: **AT BE CH DE DK ES FR GB GR IE IT LI LU MC NL PT SE**

(71) Applicant: **J. URIACH & CIA. S.A.**
**Degà Bahi, 59-67**
**E-08026 Barcelona (ES)**

(72) Inventor: **Bartroli, Javier**
**Cardenal Vives i Tuto 55**
**E-08034 Barcelona (ES)**
Inventor: **Turmo, Enric, Dr.**
**Marti i Julià 112**
**E-08903-L'Hospitalet, Barcelona (ES)**
Inventor: **Anguita, Manuel,**
**Rambla Volart 85**
**E-08026-Barcelona (ES)**
Inventor: **Carceller, Elena**
**Aragon 117**
**E-08015-Barcelona (ES)**
Inventor: **Almansa, Carmen**
**Independència 333**
**E-08026 Barcelona (ES)**

(74) Representative: **Zumstein, Fritz, Dr. et al**
**Dr. F. Zumstein**
**Dipl.-Ing. F. Klingseisen**
**Bräuhausstrasse 4**
**D-80331 München (DE)**

(54) **Pyridinium derivatives useful as PAF antagonists.**

(57) The present invention relates to novel pyridinium derivatives of formula **I**

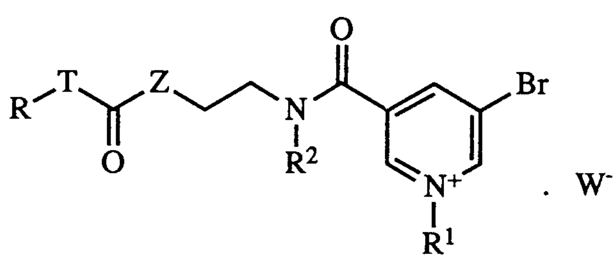

**I**

wherein $R^1$ represents $C_{1-6}$ alkyl or aryl-$C_{1-6}$ alkyl; $R^2$ represents aryl; Z is -O- or -$NR^3$-; T is -O-, -$NR^4$- or a single bond; R represents $C_{1-18}$ alkyl, $C_{1-18}$ haloalkyl, aryl, heteroaryl, aryl-$C_{1-6}$ alkyl or heteroaryl-$C_{1-6}$ alkyl, and in addition, when T represents a single bond, R can also represent a phenyl or phenylmethyl radical substituted in the para or meta position by a 2-quinolylmethoxy group; $R^3$ and $R^4$ independently represent hydrogen, $C_{1-4}$ alkyl, aryl or $C_{1-4}$ alkylcarbonyl, and in addition, $R^3$ and $R^4$ may form a ring together when Z = $NR^3$ and T = $NR^4$, or $R^3$ may be bonded to R to form a ring when Z = $NR^3$; and $W^-$ is a counter anion. These

EP 0 610 943 A1

compounds are PAF antagonists.

Field of the invention.

The present invention relates to novel pyridinium derivatives having a potent antagonist activity of the platelet activating factor (PAF). The invention also relates to a process for their preparation, to pharmaceutical compositions containing them and to their use in the treatment of diseases in which PAF is involved, such as bronchial and allergic asthma, platelet aggregation disorders, septic shock, etc, and particularly in the treatment of inflammation.

Description of the prior art.

The platelet activating factor (PAF) or (1-O-alkyl-2-acetyl-*sn*-glyceryl-3-phosphorylcholine), also called acetyl glyceryl ether phosphorylcholine (AGEPC) or PAF-acether, is a natural phospholipid synthesized by different cells (basophiles, macrophages, neutrophiles, platelets), and tissues (heart, lung and kidney) of the organism.

PAF was described for the first time as a potent platelet aggregating agent. Later on it was demonstrated to have other biological activities *in vivo*, such as peripheral vasodilatation, increase of the vascular permeability, induction of bronchoconstriction and hyperreactivity of the respiratory tract. PAF also produces immediate hypotension followed by pulmonary and renal hypertension in rats, guinea pigs, rabbits and dogs, and it has been rated as the most potent ulcerogenic agent described until now.

Furthermore, PAF has been described as being involved in inflammatory processes and it has been suggested that PAF antagonists could be useful in the treatment of some inflammatory diseases (Merlos et al., *Br. J. Pharmacol.*, **1991**,*104*,990; Kémery et al., *Arch. Dermatol. Res.*, **1989**,*281*,362)

Consequently, PAF is a mediator that is implicated in a large set of pathological processes such as asthma, septic shock, transplant rejection, thrombosis, ulceration, inflammation and renal diseases.

Even though its mechanism of action is still not known with precision, several studies show that the biological activities of PAF involve the existence of a specific receptor. Recently, the isolation of one of these receptors from human platelets has been achieved and it has been identified as a protein with a molecular weight of 160,000 daltons. On the other hand, the capacity to inhibit the binding of $^3$H-PAF to its receptors is well correlated with the amount of PAF needed to provoke the *in vitro* and *in vivo* observed effects. These facts indicate that the compounds that act as specific antagonists of PAF could result of interest for the treatment of all those pathological processes related directly or indirectly to PAF.

Until now, several PAF analogues with a potent PAF antagonist activity have been disclosed. The closest prior art, from the structural point of view, is believed to be the compounds disclosed in EP 301751, which describes PAF antagonists having the substituent depicted in Fig. 1, which also appears in the compounds of the present invention:

Fig. 1:

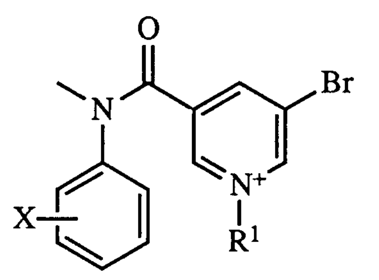

The present invention describes new compounds structurally related to those described therein, where the nature of the lipophilic chain attached to the above substituent has been substantially simplified and/or altered. In addition to their potent PAF antagonist activity, the new compounds disclosed in the present invention also show antiinflammatory activity, which makes them particularly useful for the treatment of inflammatory disorders of diverse etiology such as psoriasis, dermatitis, eczema, urticaria, etc.

Description of the invention.

The present invention relates to novel pyridinium derivatives of general formula I:

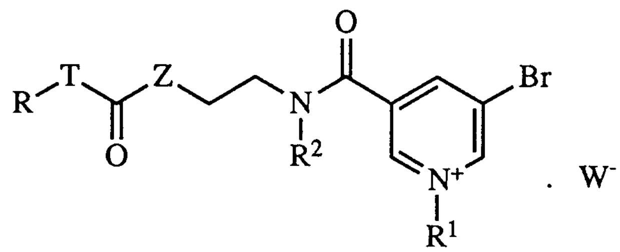

I

wherein:

$R^1$ represents a $C_{1-6}$ alkyl or aryl-$C_{1-6}$ alkyl group;

$R^2$ represents aryl;

Z is -O- or -$NR^3$-;

T is -O-, -$NR^4$- or a single bond;

R represents $C_{1-18}$ alkyl, $C_{1-18}$ haloalkyl, aryl, heteroaryl, aryl-$C_{1-6}$ alkyl or heteroaryl-$C_{1-6}$ alkyl, and in addition, when T represents a single bond, R can also represent a phenyl or phenylmethyl radical substituted in the para or meta position by a 2-quinolylmethoxy group;

$R^3$ and $R^4$ independently represent hydrogen, $C_{1-4}$ alkyl, aryl or $C_{1-4}$ alkylcarbonyl; in addition, when Z = $NR^3$ and T = $NR^4$, $R^3$ and $R^4$ may form a ring together with the group -N-C(=O)-N- to which they are bonded of formula

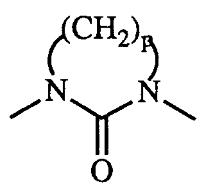

wherein p represents 2, 3 or 4, or when Z = $NR^3$, $R^3$ may be bonded to R to form a ring together with the group -T-C(=O)-N- to which $R^3$ and R are bonded of formula

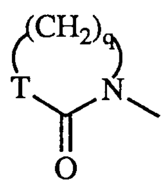

wherein q is 2, 3 or 4;

aryl in the above meanings is a phenyl group which can be optionally substituted by one or more groups chosen from halogen, trifluoromethyl, $C_{1-4}$ alkyl, $C_{1-4}$ alkoxy or hydroxy;

heteroaryl in the above meanings is any radical from an aromatic heterocycle selected from pyridine, pyrimidine, pyrazine, pyridazine, pyrrole, imidazole, triazole, furan, thiophene, thiazole, oxazole and isoxazole, and that may be optionally substituted by a group chosen from halogen, $C_{1-4}$ alkyl, $C_{1-4}$ alkoxy or hydroxy; and

$W^-$ is a counter anion;

and the solvates thereof.

The invention also provides a pharmaceutical composition which comprises an effective amount of at least one compound of formula **I** in admixture with one or more pharmaceutically acceptable excipients.

The invention further provides the use of at least one compound of formula **I** for the manufacture of a medicament for the treatment or prevention of the diseases in which PAF is involved, specially for the treatment of inflammation.

The invention still further provides a process for preparing the compounds of formula **I** which comprises reacting a compound of general formula **II**,

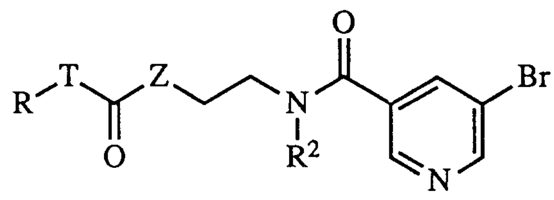

**(II)**

wherein R, $R^2$, Z and T have the previously defined meaning, with a compound of general formula $R^1$-Y (**III**, wherein $R^1$ has the previously defined meaning and Y represents a good leaving group, such as a halogen atom, methylsulfonate, *p*-toluenesulfonate) in a manner being known per se, and optionally changing the nature of the counter anion by means of ion-exchange chromatography or selective salt precipitation.

Also included in the present invention are novel compounds of formula **II**,

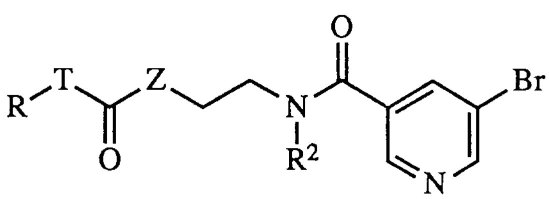

wherein R, T, Z and $R^2$ are as defined above in formula **I**. Compounds of formula **II** are useful as synthetic intermediates for making compounds of formula **I**.

In the above definitions, the term $C_{1-n}$ alkyl means, unless otherwise specified, a linear or branched alkyl group that contains from one to n carbon atoms. Therefore, in the case in which n is 3 this term includes methyl, ethyl, propyl and isopropyl. When n is 4 it includes methyl, ethyl, propyl, isopropyl, butyl, isobutyl, *sec*-butyl and *tert*-butyl. When n is 6, it includes, among others, methyl, ethyl, propyl, isopropyl, butyl, isobutyl, *sec*-butyl, *tert*-butyl, pentyl, isopentyl, neopentyl, hexyl, isohexyl, 1-methylpentyl, 2-methylpentyl, 3-methylpentyl. When n is 18 it includes, among others, methyl, ethyl, propyl, butyl, pentyl, hexyl, heptyl, octyl, nonyl, decyl, undecyl, tridecyl, tetradecyl, pentadecyl, hexadecyl, heptadecyl, octadecyl.

A $C_{1-4}$ alkoxy group means a group derived from the union of a $C_{1-4}$ alkyl group like the above mentioned to an oxygen atom of an ether functional group. Examples include methoxy, ethoxy, propoxy, isopropoxy, butoxy, isobutoxy, *sec*-butoxy, and *tert*-butoxy.

A $C_{1-4}$ alkylcarbonyl group means a group derived from the union of a $C_{1-4}$ alkyl group like the above mentioned to a carbonyl group. Examples include acetyl, propanoyl (or propionyl), butanoyl (or butyryl), 2-methylpropanoyl (or isobutyryl), pentanoyl (or valeryl), 3-methylbutanoyl (or isovaleryl), 2,2-dimethylpropanoyl (or pivaloyl).

The term halogen means fluorine, chlorine, bromine or iodine.

A $C_{1-18}$ haloalkyl group means a group resulting from the substitution of one or more hydrogen atoms of a $C_{1-18}$ alkyl group like the above mentioned by one or more halogen atoms, which can be the same or different. Examples include trifluoromethyl, fluoromethyl, chloroethyl, fluoroethyl, iodoethyl, pentafluoroethyl, fluoropropyl, chloropropyl, 2,2,3,3,3-pentafluoropropyl, 2,2,3,3-tetrafluoropropyl, heptafluoropropyl, etc.

The term aryl represents a phenyl group or a phenyl group substituted by one or more groups chosen from halogen, trifluoromethyl, $C_{1-4}$ alkyl, $C_{1-4}$ alkoxy or hydroxy. Examples include: phenyl, 2-methylphenyl, 4-methylphenyl, 4-chlorophenyl, 4-bromophenyl, 4-methoxyphenyl, 2-methoxyphenyl, 4-trifluoromethylphenyl.

The term aryl-$C_{1-6}$-alkyl represents a group resulting from the substitution of one hydrogen atom of a $C_{1-6}$ alkyl group like the above mentioned by an aryl group like the above defined. Examples include, among others, benzyl, (4-trifluoromethylphenyl)methyl, (4-fluorophenyl)methyl, 2-phenylethyl, 2-(4-trifluormethylphenyl)ethyl, 3-phenylpropyl, 4-phenylbutyl, 5-phenylpentyl, 6-phenylhexyl, etc.

The term heteroaryl represents any radical from an aromatic heterocycle selected from the group consisting of pyridine, pyrimidine, pyrazine, pyridazine, pyrrole, imidazole, triazole, furane, thiophene, thiazole, oxazole and isoxazole. Said heterocycles may be optionally substituted by a group chosen from halogen, $C_{1-4}$ alkyl, $C_{1-4}$ alkoxy or hydroxy.

The term heteroaryl-$C_{1-6}$-alkyl represents a group resulting from the substitution of one hydrogen atom of a $C_{1-6}$ alkyl group like the above mentioned by a heteroaryl group like the above defined. Examples include, among others, 2-pyridylmethyl, 3-pyridylmethyl, 4-pyridylmethyl, 2-pyrazinylmethyl, 2-furylmethyl, 3-furylmethyl, 2-pyrimidinylmethyl, 4-pyrimidinylmethyl, 3-pyridazinylmethyl, 4-pyridazinylmethyl, 2-pyrrolylmethyl, 3-pyrrolylmethyl, 1-(3-pyridyl)ethyl, 2-(3-pyridyl)ethyl, 3-(3-pyridyl)propyl.

Examples of the counter anion W- include pharmaceutically acceptable anions, for example: anions of an inorganic acid such as chloride, bromide, iodide, nitrate, sulfate, phosphate, etc; anions of an organic acid, such as methanesulfonate, trifluoromethanesulfonate, ethanesulfonate, benzenesulfonate, p-toluenesulfonate, acetate, fumarate, oxalate, citrate, maleate; and anions of other mineral and carboxylic acids well known to those skilled in the art.

Of the above groups which may be represented by $R^1$, $C_{1-6}$ alkyl is preferred, and $C_{1-3}$ alkyl is more preferred.

Preferred meanings for the substituent R are $C_{1-18}$ alkyl, $C_{1-18}$ haloalkyl, aryl and aryl-$C_{1-6}$ alkyl. In addition, when T represents a single bond, other preferred meanings for R are phenyl or phenylmethyl substituted in the para or meta position by a 2-quinolylmethoxy group.

Preferred embodiments of the present invention are those compounds of formula **I** where:

$R^1$ represents $C_{1-3}$ alkyl; and

$R^2$, R, T, Z and $W^-$ have the previously defined meaning.

More preferred embodiments of the present invention are those compounds of formula **I** where:

$R^1$ represents $C_{1-3}$ alkyl;

$R^2$ represents Ph; and

R, T, Z and $W^-$ have the previously defined meaning.

Most preferred embodiments of the present invention are the following groups of compounds:

I) Those compounds of formula **I** where:

$R^1$ represents $C_{1-3}$ alkyl;

$R^2$ represents Ph;

R represents $C_{1-18}$ alkyl, $C_{1-18}$ haloalkyl, aryl, heteroaryl, aryl-$C_{1-6}$ alkyl or heteroaryl-$C_{1-6}$ alkyl;

Z is -$NR^3$- and T represents -O- or a single bond, or Z is -O- and T represents -$NR^4$-; and

$W^-$, $R^3$ and $R^4$ have the previously defined meaning.

II) Those compounds of formula **I** where:

$R^1$ represents $C_{1-3}$ alkyl;

$R^2$ represents Ph;

R represents a phenyl or phenylmethyl radical substituted in the para or meta position by a 2-quinolylmethoxy group;

T represents a single bond;

Z is -$NR^3$-; and

$W^-$ and $R^3$ have the previously defined meaning.

The formulae of some specific compounds are represented below, together with the number corresponding to the example in which their preparation is described:

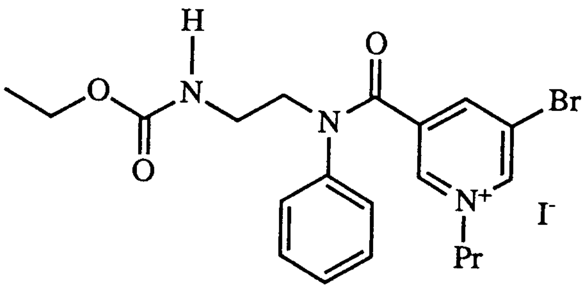

**1**

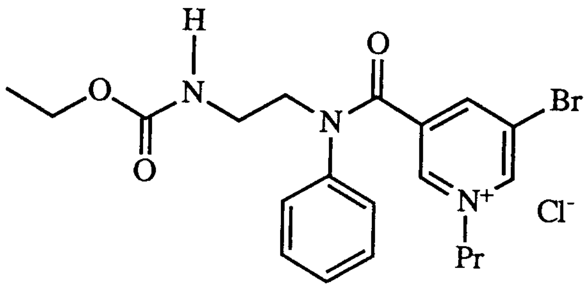

**2**

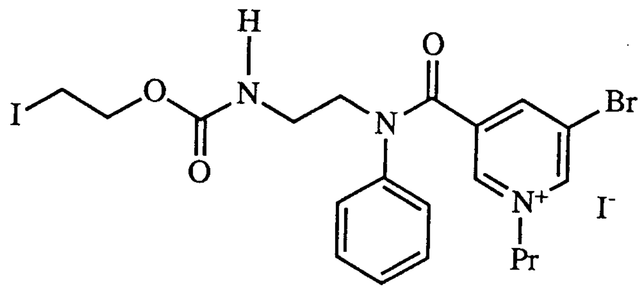

**3**

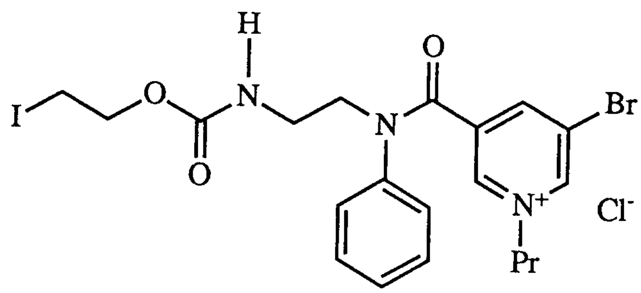

**4**

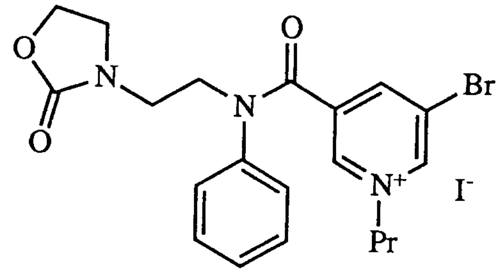

**5**

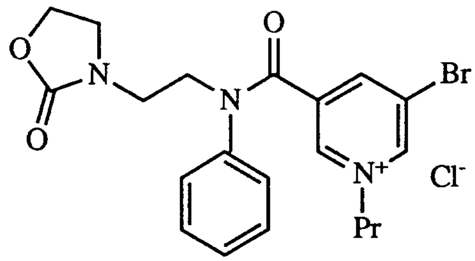

6

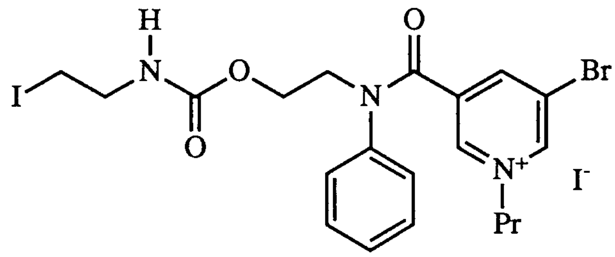

7

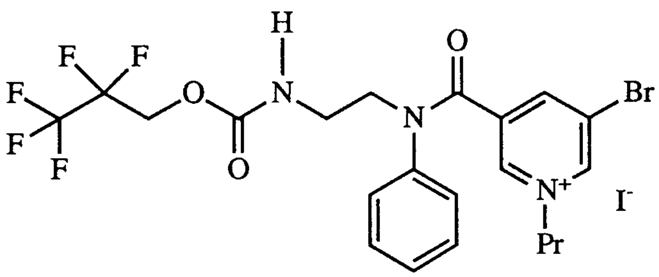

8

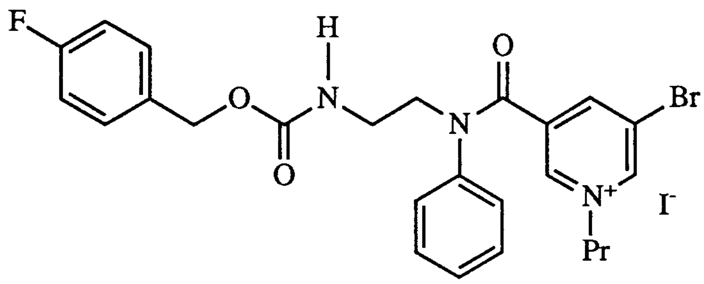

9

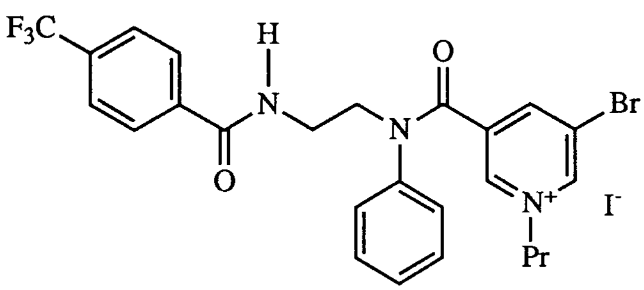

10

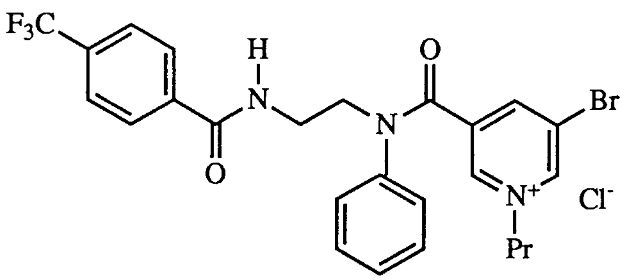

11

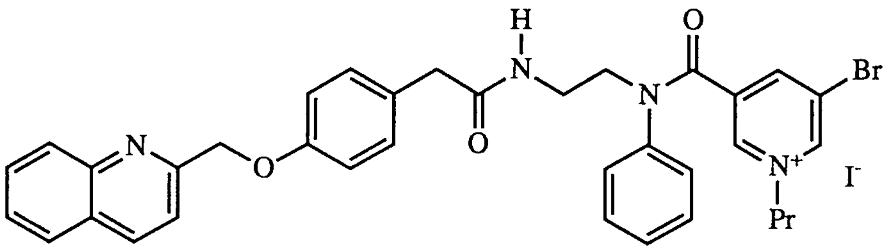

12

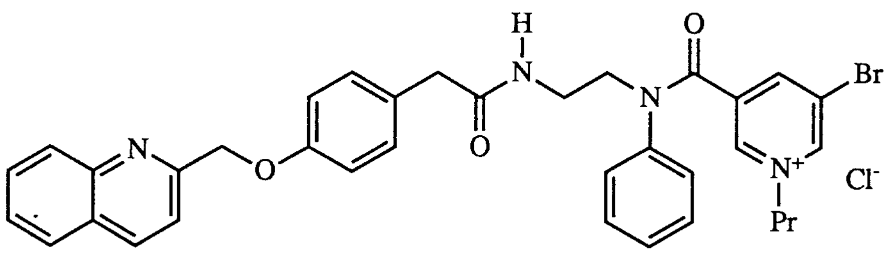

13

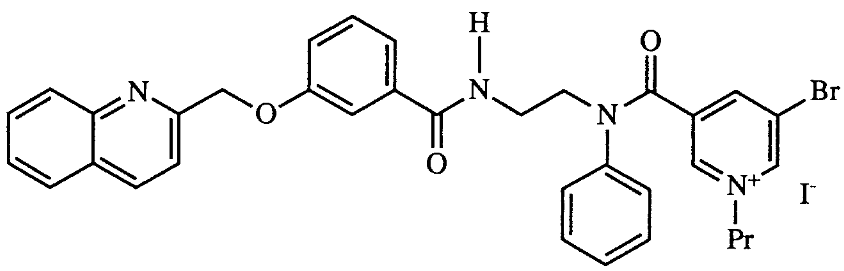

14

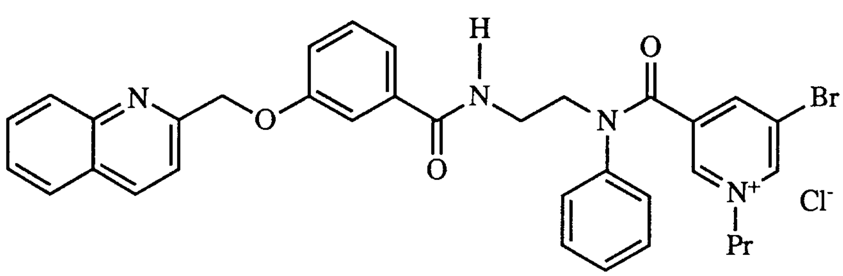

15

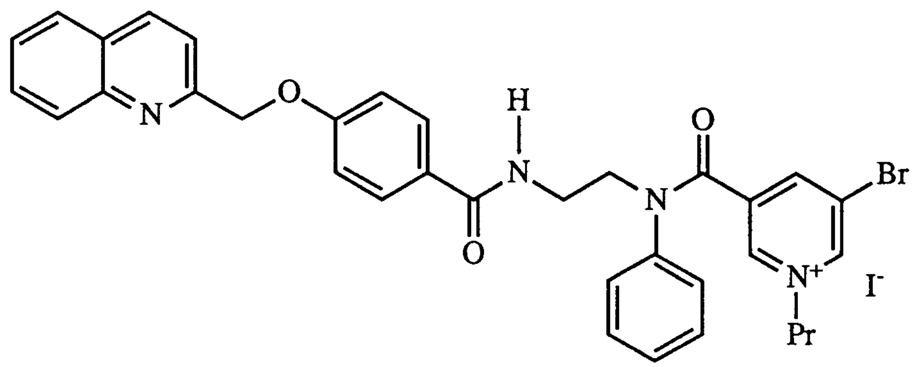

16

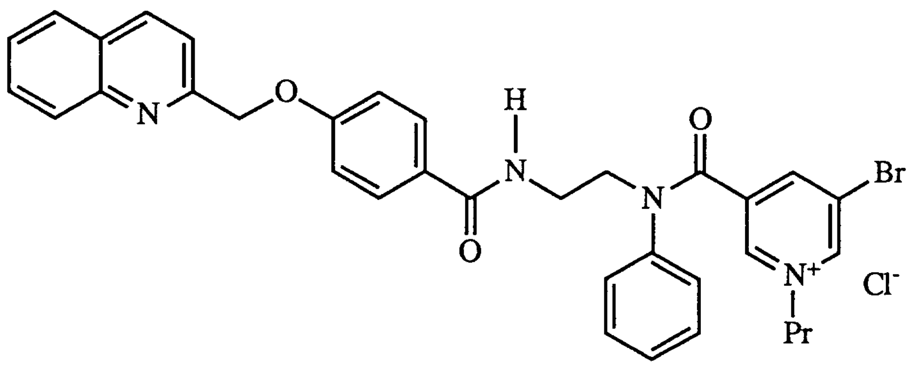

17

The compounds of the present invention can exist in unsolvated as well as solvated forms, including hydrated forms. In general, the solvated forms, with pharmaceutically acceptable solvents such as water, ethanol and the like, are equivalent to the unsolvated forms for purposes of the invention.

Some compounds of the present invention can exist as different diastereoisomers and/or optical isomers because of the existence of chiral centres in the molecule. Diastereoisomers can be separated by conventional techniques such as chromatography or fractional crystallization. The optical isomers can be resolved using any of the conventional optical resolution techniques to give optically pure isomers. Such a resolution can be performed in any chiral synthetic intermediate as well as in the products of general formula **I**. The optically pure isomers can also be individually obtained using enantiospecific synthesis. The present invention covers the individual isomers as well as their mixtures (e.g. racemic mixtures), whether as obtained by synthesis or by physically mixing them up.

The invention also provides processes for preparing the compounds of formula **I**. The precise method used for the preparation of a given compound of the present invention may vary depending on its chemical structure. Scheme 1 illustrates the general method for their preparation.

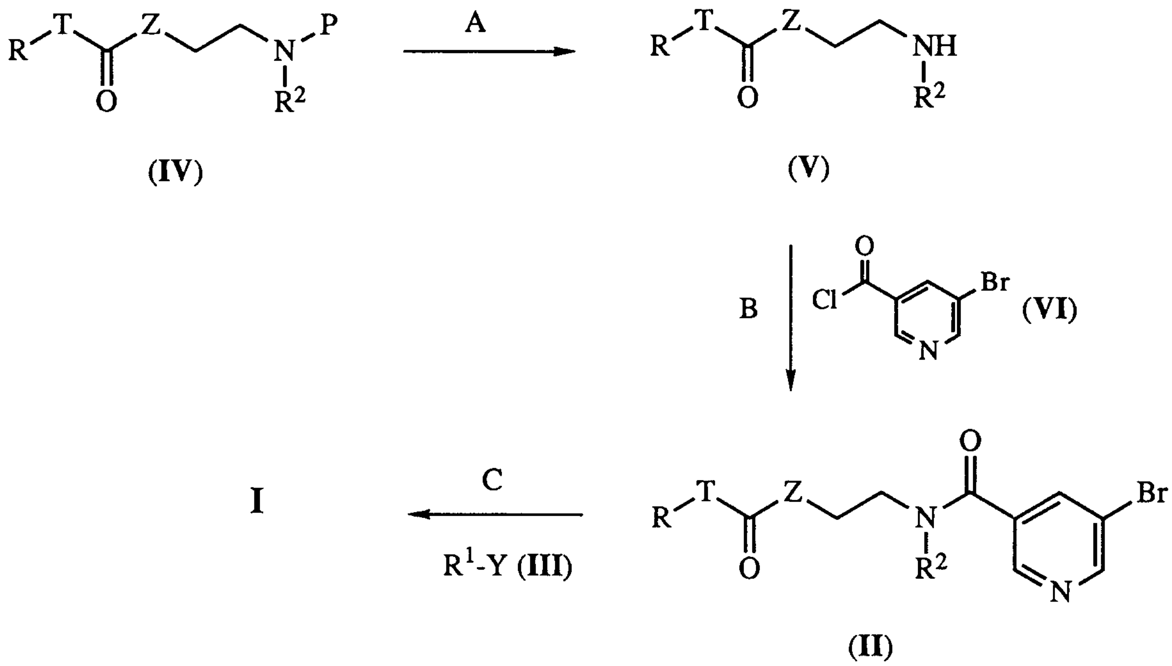


(IV) (V) (VI) I (II)

Scheme 1

Wherein:

R, $R^1{}_1$ $R^2$, T and Z have the previously defined meaning;

Y represents a good leaving group such as a halogen atom, methylsulfonate, *p*-toluenesulfonate; and

P means an amine protecting group such as a *tert*-butoxycarbonyl group.

In Step A, compounds of formula **V** are prepared from compounds of formula **IV** by deprotection of the nitrogen to give the free amine. The reagents and the reaction conditions needed will depend on the nature of the protecting group used. Thus, if the protecting group is *tert*-butoxycarbonyl, deprotection can be carried out by treatment with an acid (e.g. an inorganic acid such as hydrochloric acid, phosphoric acid, sulfuric acid, etc. or an organic acid such as toluenesulfonic acid, methanesulfonic acid, acetic acid, etc.) in a suitable solvent such as water, alcohol, tetrahydrofuran or dioxane, at a temperature between 0 and 150 ° C and during a period of time from 30 min. to 3 h.

The reaction of a compound of formula **V** (Step B) with 5-bromonicotinoyl chloride (**VI**) in the presence of a proton scavenger amine, such as pyridine or triethylamine, in a suitable solvent, for example dichloromethane or chloroform, or the same proton scavenger amine can be used as solvent, leads to the compounds of general formula **II**. The reaction is carried out at a temperature between 0 ° C and that of the boiling point of the solvent, during a period of time from 30 min to 24 h. The compounds thus obtained are purified by conventional methods such as flash chromatography or recrystallization. As an alternative to the acid chloride **VI**, the anhydride can be employed. Alternatively, compounds of formula **II** may be prepared by a dehydration procedure between amines **V** and 5-bromonicotinic acid. This process can be carried out by using any conventional reaction of amide bond formation, such as reacting an amine with an acid in the presence of dicyclohexylcarbodiimide and 1-hydroxybenzotriazole in a suitable solvent like dioxane, tetrahydrofuran, acetonitrile, chloroform or N,N-dimethylformamide, at a temperature ranging from 0 to 60 ° C.

Finally, the reaction of a compound of formula **II** (Step C) with a compound of formula $R^1$-Y (**III**) leads to the compounds of general formula **I**. The reaction can be carried out without solvent in the case where **III** is liquid and non-volatile, or in the presence of a solvent when **III** is solid or very volatile, but in either case an excess of reagent is always used. The suitable solvents are preferentially those with high polarity, such as acetonitrile, tetrahydrofuran, dioxane, N,N-dimethylformamide or dimethylsulfoxide. The reaction is carried out at a temperature between room temperature and 120 ° C; the reaction time will depend mainly on the nature of the reagent $R^1$-Y and the temperature used, but a period between 1 hour and 72 h will usually suffice. The desired compound can be isolated by concentration of the reaction residue or by precipitation with a less polar solvent. The compound obtained in this way is usually pure enough. However, if that is not the case, it can be purified by conventional techniques such as flash chromatography or recrystallization.

The compounds of formula **I** thus obtained are salts in which the anion comes from the reagent $R^1$-Y. If desired, such anion can be changed by using an ionic interchange resin or by selective salt precipitation.

The preparation of the compounds of formula **IV** may vary depending on its chemical structure; scheme 2 illustrates the general method for their preparation:

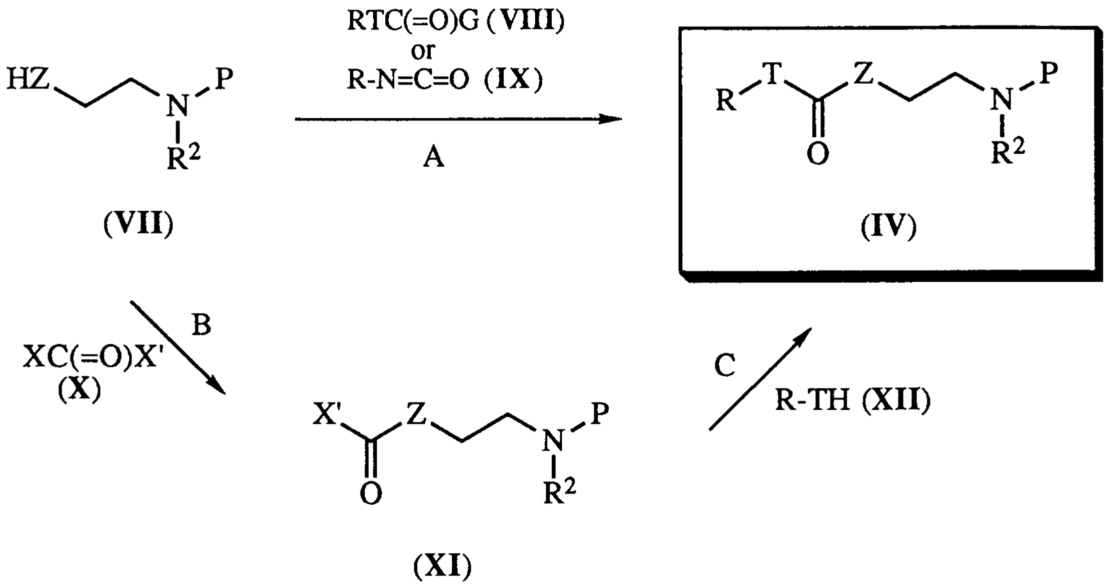


(VII)

(IV)

(XI)

Scheme 2

Wherein:

R, $R^2$, Z, T and P have the previously defined meaning;

G means a halogen atom, such as chlorine, or -OPh;

XC( = O)X' represents a reagent of the so-called phosgene equivalents, that is to say a doubly activated carbonyl group, such as phenylchloroformate (X = Cl, X' = OPh).

Compounds of formula **IV** are prepared from compounds of formula **VII**, which either are known compounds (see for example EP 301751) or, if they have not been described, they can be prepared following analogous methods to those described in the literature. Thus, a compound of formula **VII** (Step A) is allowed to react with a compound of formula RTC( = O)G (**VIII**, wherein R, T and G have the previously defined meaning) or a compound of formula R-N = C = O (**IX**, wherein R has the previously defined meaning) in a suitable solvent. Examples of suitable solvents include: halogenated hydrocarbons, such as dichloromethane and chloroform; ethers, such as diethylether, tetrahydrofuran and dioxane; and aromatic hydrocarbons, such as benzene and toluene. The reaction is carried out in the presence of a base, which can also be used as solvent. Examples of suitable bases include triethylamine and pyridine. The reaction takes place at a temperature between 0 and 100 ° C; the time required for the reaction may vary depending on the temperature and the nature of the reagents and the base employed, but in general a period of from 30 min to 24 h will usually suffice.

Alternatively, when T represents O or $NR^4$, the compounds of formula **IV** can also be prepared by reaction of a compound of formula **VII** (Step B) with a reagent (**X**) of the so-called phospgene equivalents, i.e. a doubly activated carbonyl group, wherein X and X' represent leaving groups which can be identical or different. Although in principle any phosgene equivalent described in the literature could be employed, we have found that phenyl chloroformate (X = Cl, X' = OPh) works conveniently. The reaction is carried out in a solvent in the presence of a proton scavenger base. Examples of these are the same that have been mentioned above for step A of scheme 2. The experimental conditions (temperature and reaction time) are similar to those described in step A. Finally, the intermediate thus obtained **XI** (Step C) is allowed to react with a compound of formula R-TH (**XII**). The reaction is conducted under conditions similar to those described for step B.

The compounds of formula **IV** wherein T represents a bond may also be obtained by a dehydration procedure between a compound of formula **VII** and a carboxylic acid of formula RCOOH, wherein R has the previously defined meaning. This dehydration process can be carried out by using any conventional

reaction of ester or amide bond formation.

These reactions are all per se known ones and can be carried out in accordance with known conditions.

The compounds of formulae **VIII**, **IX**, **XII** and RCOOH are either commercial, or widely described in the literature or can be prepared by methods similar to those already described, starting from commercially available products.

Compounds of general formula **I**, being potent PAF antagonists, are useful as preventive and therapeutic drugs for the treatment of circulatory diseases caused by PAF, such as thrombosis, cerebral apoplexy (e.g. cerebral hemorrhage, cerebral thrombosis), myocardial infarction, angina pectoris, thrombotic phlebitis, thrombocytopenic purpura; nephritis (e.g. glomerular nephritis), diabetic nephrosis, pancreatitis; shock states (e.g. septic shock observed after severe infection or postoperatively, intravascular agglutination syndrome caussed by endotoxin, anaphylactic shock, hemorrhagic shock, myocardial ischemia); gastrointestinal tract diseases where PAF is involved (e.g. gastric ulcer, inflammatory bowel disease); asthma and other diseases related to allergy; pneumonia; rejection due to increased PAF production after implantation of organs; and postoperative organodysfunctions (e.g. in heart, liver and kidney); central nervous system related disorders (e.g. multiple sclerosis) and particularly for the treatment of inflammatory disorders (e.g. psoriasis, dermatitis, eczema, urticaria, pruritus, and sunburns). They can also be used for contraception of female mammals by suppressing cell division and/or ovoimplantation on the uterus, in the treatment of endometriosis and in the prevention or treatment of hyperendothelinemia induced by excess secretion of endothelin.

According to the activity of the compounds disclosed, the present invention further provides compositions that contain one or more compounds of the present invention, together with an excipient and optionally other auxiliary agents, if necessary. The compounds of the present invention can be administered in different pharmaceutical preparations, the precise nature of which will depend, as it is well known, upon the chosen route of administration and the nature of the pathology to be treated.

Thus, solid compositions according to the present invention for oral administration include compressed tablets, dispersible powders, granules and capsules. In tablets, one or more of the active component(s) is admixed with at least one inert diluent such as lactose, starch, mannitol, microcrystalline cellulose or calcium phosphate; granulating and disintegrating agents for example corn starch, gelatine, microcrystalline cellulose or polyvinylpyrrolidone; and lubricating agents for example magnesium stearate, stearic acid or talc. The tablets may be coated by known techniques to delay disintegration and absorption in the gastrointestinal tract and, thereby, provide a sustained action over a longer period. Gastric film-coated or enteric film-coated can be made with sugar, gelatin, hydroxypropylcellulose, or acrylic resins. Tablets with a sustained action may also be obtained using an excipient which provides regressive osmosis, such as the galacturonic acid polymers. Formulations for oral use may also be presented as hard capsules of absorbable material, such as gelatin, wherein the active ingredient is mixed with an inert solid diluent and lubricating agents, or pasty materials, such as ethoxylated saturated glycerides. Soft gelatin capsules are possible wherein the active ingredient is mixed with water or an oil medium, for example peanut oil, liquid paraffin or olive oil.

Dispersible powders and granules suitable for preparation of a suspension by the addition of water provide the active ingredient in admixture with a dispersing or wetting agent, a suspending agent, such as sodium carboxymethylcellulose, sodium alginate, polyvinylpirrolidone, gum tragacanth, xantham gum, gum acacia, and one or more preservatives, such as methyl or *n*-propyl-*p*-hydroxybenzoate. Additional excipients, for example sweetening, flavoring and coloring agents may also be present.

Liquid compositions for oral administration include emulsions, solutions, suspensions, syrups and elixirs containing commonly used inert diluents, such as distilled water, ethanol, sorbitol, glycerol, or propylene glycol. Such compositions may also comprise adjuvants such as wetting agents, suspending agents, sweetening, flavoring, perfuming, preserving agents and buffers.

Other compositions for oral administration include spray compositions, which may be prepared by known methods and which comprise one or more active compound(s). The spray compositions will contain a suitable propellent.

Preparations for injection according to the present invention for parenteral administration include sterile aqueous or non-aqueous solutions, suspensions or emulsions, in a non-toxic parentally-acceptable diluent or solvent. Examples of aqueous solvents or suspending media are distilled water for injection, the Ringer's solution, and isotonic sodium chloride solution. Examples of non-aqueous solvents or suspending media are propylene glycol, polyethylene glycol, vegetable oils such as olive oil, or alcohols such as ethanol. These compositions may also include adjuvants such as wetting, preserving, emulsifying and dispersing agents. They may be sterilized by one of the known methods or manufactured in the form of sterile solid compositions which can be dissolved in sterile water or some other sterile injectable medium immediately

before use. When all of the components are sterile, the injectables will maintain the sterility if they are manufactured in sterile environment.

When the compound is administered by intravenous injection, for example for prevention and treatment of shock, the compound can be given as a bolus injection or by infusion.

A compound of the invention may also administered in the form of suppositories for rectal administration of the drug, or as creams, ointments, pastes, lotions, gels, sprays, foams, aerosols, solutions, suspensions or powders for topical use and pessaries for vaginal administration. Such compositions are prepared following conventional procedures well known to those skilled in the art.

The dosage and frequency of dose may vary depending upon symptoms, age and body weight of the patient, as well as upon the nature of the disease to be treated and the route of administration. In general, the compounds of the invention may be administered orally or intravenously in a daily dose of from 1-1000 mg for an adult, preferably a dosage from 5-250 mg, which may be administered either as a single dose or as divided doses. A preferred dosage for human patients is from 0.005 to 5 mg/kg of body weight, more preferably from 0.05 to 1 mg/kg of body weight.

The compositions for topical administration will contain 0.5-10% by weight of a compound of formula **I**.

Following are some representative preparations for tablets, capsules, syrups, aerosols, injectables and creams. They can be prepared following standard procedures and they are useful in the treatment of diseases where PAF is involved.

| **Tablets** | |
|---|---|
| Compound of formula **I** | 100 mg |
| Dibasic calcium phosphate | 125 mg |
| Sodium starch glycolate | 10 mg |
| Talc | 12.5 mg |
| Magnesium stearate | 2.5 mg |
| | 250.0 mg |

| **Hard gelatin capsules** | |
|---|---|
| Compound of formula **I** | 100 mg |
| Lactose | 197 mg |
| Magnesium stearate | 3 mg |
| | 300 mg |

| **Syrup** | |
|---|---|
| Compound of formula **I** | 0.4 g |
| Sucrose | 45 g |
| Flavouring agent | 0.2 g |
| Sweetening agent | 0.1 g |
| Water to | 100 ml |

| **Aerosol** | |
|---|---|
| Compound of formula **I** | 4 g |
| Flavouring agent | 0.2 g |
| Propylene glycol to | 100 ml |
| Suitable propellent to | 1 unit |

| **Injectable preparation** | |
|---|---|
| Compound of formula **I** | 100 mg |
| Benzylic alcohol | 0.05 ml |
| Propylene glycol | 1 ml |
| Water to | 5 ml |

| **Cream** | |
|---|---|
| Compound of formula **I** | 2 g |
| Dimethyl acetamide | 2 g |
| White paraffin | 25 g |
| Stearic alcohol | 22 g |
| Propylene glycol | 12 g |
| Sodium lauryl sulfate | 1.5 g |
| Methylparabene | 0.3 g |
| Purified water | 31.6 g |

The following pharmacological tests explain the activity of the compounds of the present invention in more detail.

**PHARMACOLOGICAL TEST 1**

Inhibition of platelet aggregation induced by PAF

Blood wass obtained by cardiac puncture of male New Zealand rabbits (b.w. 2-2.5 kg) and coagulation was prevented by adding 1 volume of 3.16% sodium citrate dihydrate in 9 volumes of blood. Platelet-rich plasma (PRP) was prepared by centrifuging the blood at 250xg for 10 min at 4°C and then it was diluted with platelet-poor plasma (PPP) obtained by further centrifuging at 3000xg for 10 min. The platelet count was adjusted to $3x10^5/mm^3$. Platelet aggregation induced by PAF ($C_{18}$, prepared in our laboratory) (15 nM) was determined by the Born nephelometric technique (*J. Physiol.*, **1962**,*162, 670*) using an aggregometer Chrono-log 500. The activities of the inhibitors were expressed as the $IC_{50}$ value, that is to say the concentration of the drug needed to inhibit platelet aggregation by 50%. The results are shown in table **I** below.

TABLE I

| Compound Nº | $IC_{50}$ (μM) |
|---|---|
| 1 | 0.018 |
| 2 | 0.0042 |
| 3 | 0.010 |
| 4 | 0.016 |
| 5 | 0.036 |
| 7 | 0.0021 |
| 8 | 0.0042 |
| 9 | 0.0038 |
| 10 | 0.0045 |
| 11 | 0.023 |
| 13 | 0.052 |

**PHARMACOLOGICAL TEST 2**

Inhibition of the hypotensive effect induced by PAF in normotensive rats.

Male Sprague Dawley rats (b.w. 180-220 g) anaesthetized with sodium pentobarbital (50 mg/kg, i.p. 1mL/100 g) were used. In order to measure the arterial pressure, a polyethylene catheter was introduced into the carotid artery. The arterial pressure was recorded with the help of a transducer coupled to a R611 Beckman polygraph. The test compounds were administered through the femoral vein 3 min before PAF injection (0.5 mcg/kg, i.v.). Control animals received only the vehicle. Table II shows the inhibition of PAF-induced hypotension of the different compounds, expressed as $ID_{50}$ values, that is to say, the amount of compound by weight of animal (dose) needed to inhibit PAF-induced hypotension by 50%.

TABLE II

| Compound Nº | $ID_{50}$ (mg/kg i.v.) |
|---|---|
| 1 | 0.046 |
| 2 | 0.039 |
| 5 | 0.16 |
| 7 | <0.025 |
| 9 | 0.079 |
| 11 | 0.022 |
| 13 | 0.0065 |

**PHARMACOLOGICAL TEST 3**

Rodent ear oedema induced by application of several irritant agents

This test was performed according to the method described by Young et al. (*Pharmacological Methods in the Control of Inflammation*., ed. Chang, J.Y. & Lewis, A.J. pp 215-231. New York: Alan R. Liss, 1989). Groups of 8-10 Swiss, male mice, 6-8 weeks old, were used in the experiments. Animals had free access to food and water. Irritant dermatitis was elicited by painting both faces of the left ear of the mouse with 20 µL (10 µL each face) of the inflammatory agents. Concentrations (w/v) of the irritants were: 10% arachidonic acid and 0.01% TPA (12-O-tetradecanoylphorbol 13-acetate), both dissolved in acetone. Total applied doses were 2 and 0.002 mg/ear, respectively. Treatment was effected by application of a total of 20 µL of the test compound dissolved in acetone to both faces of the left ear of the mouse 30 min before the application of the irritant agent. Control animals received only the vehicle. At 1 h (arachidonic acid) and 6 h (TPA) after administration of the irritant, animals were killed by cervical dislocation. An 8 mm diameter punch biopsy was performed on each ear, the right (untreated) ear being the control for each animal. The swelling induced by the irritant was quantified as the percentage increase in the weight of the left ear biopsy over that of the right ear biopsy. Percentage inhibition of oedema was calculated according to the following formula:

$$\%\ \text{inhibition} = \left(1 - \frac{\text{wt. of inflamed-treated ear - wt. of control ear}}{\text{wt. of inflamed ear - wt. of control ear}}\right) \times 100$$

Activity of the inhibitors was expressed as the $ID_{50}$ value, i.e. the amount of compound by weight of animal (dose) required to inhibit the increase in weight by 50%, with respect to control animals. Results are shown in the following table:

| Compound No | Arachidonic acid | TPA |
|---|---|---|
| | ID50 (mg/ear) | |
| 1 | 0.31 | 0.31 |
| 3 | 0.3-1 | 0.76 |
| 5 | ≧1 | 0.68 |
| 7 | 0.50 | 0.20 |
| 8 | ≈1 | 0.3-1 |
| 9 | ≈1 | 0.3-1 |
| 10 | ≈1 | 0.3-1 |
| 13 | >0.3 | >0.1 |
| 15 | - | ≦0.1 |
| 17 | - | <0.1 |

The following examples illustrate, but do not limit, the scope of the invention:

REFERENCE EXAMPLE 1

N-*tert*-butoxycarbonyl-N-phenyl-N'-(ethoxycarbonyl)ethylendiamine

To a solution of N-*tert*-butoxycarbonyl-N-phenylethylendiamine (1 g, 4.23 mmol) (obtained according to EP 301751) in $CHCl_3$ (15 mL) was added triethylamine (1.17 mL, 8.46 mmol) and the resulting mixture was cooled to 0 °C. Ethyl chloroformate (0.44 mL, 4.65 mmol) was added and the reaction mixture was stirred at 0 °C for 1 h. More $CHCl_3$ was added and the mixture was washed with 10% $NaHCO_3$ solution, water and brine. The organic phase was dried and the solvent was removed to yield an oil that was purified by chromatography on silica gel (hexane-ethyl acetate, 1:1). 0.77 g of the desired product was obtained (yield: 59%).
IR (KBr) ν: 3339, 2972, 2927,1692, 1522, 1491, 1388, 1364, 1250, 1149, 1039, 765, 697 $cm^{-1}$;
$^1$H-NMR (80 MHz, $CDCl_3$) δ (TMS): 7.24 (m, 5H, Ar), 5.01 (broad s., 1H, NH), 4.09 (q, J = 7.1Hz, 2H, $OCH_2$), 3.77 (t, J = 6Hz, 2H, $\underline{CH_2}CH_2NH$), 3.34 (broad q, J = 6Hz, 2H, $CH_2\underline{CH_2}NH$), 1.42 (s, 9H, $CH_3$), 1.21 (t, J = 7.1Hz, 3H, $OCH_2\underline{CH_3}$).

REFERENCE EXAMPLE 2

N-phenyl-N'-ethoxycarbonylethylendiamine

To a solution of the product obtained in reference example 1(0.76 g, 2.45 mmol) in MeOH (5 mL) and $CHCl_3$ (5 mL) was added 10 mL of a saturated solution of HCl in MeOH and the reaction mixture was heated at 50 °C for 2 h. The resulting solution was concentrated and washed with 10% $NaHCO_3$ solution. The organic phase was dried and the solvent was removed to afford 0.5 g of a colourless oil (yield: 97%).
IR (KBr) ν: 3376, 2974, 2930, 1693, 1599, 1504, 1254, 1035, 750, 693 $cm^{-1}$;
$^1$H-NMR (80 MHz, $CDCl_3$) δ (TMS): 7.18 (m, 2H, Ar), 6.66 (m, 3H, Ar), 4.92 (broad s., 1H, NHCO), 4.13 (q, J = 7Hz, 2H, $OCH_2$), 3.33 (m, 5H, $CH_2CH_2$ + NHAr), 1.24 (t, J = 7Hz, 3H, $OCH_2\underline{CH_3}$).

REFERENCE EXAMPLE 3

5-Bromo-3-[N-(2-ethoxcarbonlamino)ethyl-N-phenl]carbamoylpyridine

To a solution of the product obtained in reference example 2(0.47 g, 2.44 mmol) in $CHCl_3$ (15 mL), was added triethylamine (1.02 mL, 7.33 mmol) and the resulting mixture was cooled to 0 °C. Then, 5-bromonicotinic acid chloride (691 mg, 2.68 mmol) was added and the reaction mixture was stirred at 0 °C for 15 min and then at 25 °C for 30 min. Chloroform was added, then 10% $NaHCO_3$ solution and the solution was washed with brine. The organic layer was dried over anhydrous sodium sulfate, filtered and concentrated to afford an oil that was purified by chromatography on silica gel (ethyl acetate-hexane, 3:1). 910 mg of the desired product was obtained (yield: 95%).
IR (KBr) ν: 3700-3200, 3052, 2974, 2930, 1702, 1641, 1589, 1522, 1488, 1385, 1251, 891, 747,700 $cm^{-1}$;
$^1$H-NMR (80 MHz, $CDCl_3$) δ (TMS): 8.52 (broad s, 1H, pyr), 8.32 (broad s, 1H, pyr), 7.81 (m, 1H, pyr) 7.4-7.0

(m, 5H, Ar), 5.17 (broad s., 1H, NH), 4.10 (q, J= 7.2Hz, 2H, $OCH_2$), 4.08 (t, J= 6Hz, 2H, $\underline{CH_2}CH_2NH$), 3.47 (broad q, J= 6Hz, 2H, $CH_2\underline{CH_2}NH$), 1.22 (t, J= 7.2Hz, 3H, $OCH_2\underline{CH_3}$).

REFERENCE EXAMPLE 4

N-*tert*-butoxycarbonyl-N-phenyl-N'-(2-chloroethoxycarbonyl)ethylendiamine

Following the procedure described in reference example 1, but using 2-chloroethyl chloroformate instead of ethyl chloroformate, the title compound of this example was obtained.
IR (KBr) $\nu$: 3334, 2970, 1691, 1518, 1491, 1389, 1363, 1246, 1149, 765, 698 $cm^{-1}$;
$^1$H-NMR (80 MHz, $CDCl_3$) $\delta$ (TMS): 7.28 (m, 5H, Ar), 5.22 (broad s., 1H, NH), 4.32 (t, J= 5.8Hz, 2H, $CH_2$), 3.9-3.6 (m, 4H), 3.39 (broad q, J= 5.6Hz, 2H, $CH_2\underline{CH_2}NH$), 1.45 (s, 9H, $CH_3$).

REFERENCE EXAMPLE 5

N-phenyl-N'-(2-chloroethoxycarbonyl)ethylendiamine

Following the procedure described in reference example 2, but starting from the compound obtained in reference example 4, the title compound of this example was obtained.
mp: 29-32 °C;
IR (KBr)$\nu$: 3344, 3049, 2942, 1698, 1599, 1503, 1248, 1132, 750, 692 $cm^{-1}$;
$^1$H-NMR (80 MHz, $CDCl_3$) $\delta$ (TMS): 7.18 (m, 2H, Ar), 6.66 (m, 3H, Ar), 5.07 (broad s., 1H, NHCO), 4.33 (t, J= 5.2Hz, 2H, $CH_2$), 3.65 (t, J= 5.2Hz, 2H, $CH_2$), 3.6-3.2 (m, 5H, $CH_2CH_2$ + NHAr).

REFERENCE EXAMPLE 6

5-Bromo-3-[N-[2-(2-chloroethoxy)cabonylamino]ethyl-N-phenyl]carbamoylpyridine

Following the procedure described in reference example 3, but starting from the compound obtained in reference example 5, the title compound of this example was obtained.
IR (KBr) $\nu$: 3325, 3053, 2938, 1715, 1638, 1488, 1388, 1296, 1246, 1019, 892, 747, 700 $cm^{-1}$;
$^1$H-NMR (80 MHz, $CDCl_3$) $\delta$ (TMS): 8.51 (d, J= 2.1Hz, 1H, pyr), 8.31 (d, J= 1.7Hz, 1H, pyr), 7.82 (m, 1H, pyr), 7.4-7.0 (m, 5H, Ar), 5.36 (broad s., 1H, NH), 4.30 (t, J= 5.6Hz, 2H, $CH_2$), 4.11 (t, J= 5.9Hz, 2H, $CH_2$), 3.65 (t, J= 5.6Hz, 2H, $CH_2$), 3.49 (broad q, J= 5.9Hz, 2H, $\underline{CH_2}NH$).

REFERENCE EXAMPLE 7

5-Bromo-3-[N-[2-(2-oxo-1,3-oxazolidin-3-yl)]ethyl-N-phenyl]carbamoylpyridine

To a solution of the compound obtained in reference example 6 (0.75 g, 1.75 mmol) in 100 mL of methyl ethyl ketone, was added potassium carbonate (486 mg, 3.5 mmol) and potassium iodide (2.92 g, 17.5 mmol) and the reaction mixture was heated at reflux for 3 days. The solvent was removed and the residue was partitioned between water and chloroform. The organic layer was separated, dried over $MgSO_4$ and the solvent was removed to yield 1.10 g of a residue that was purified by chromatography on silica gel eluting with ethyl acetate. 0.55 g of the title compound of this example was obtained (yield: 80%).
mp: 95-96 °C;
IR (KBr) $\nu$: 3600-3200 ($H_2O$), 3027, 2985, 2943, 1723, 1652, 1488, 1384, 1295, 1268, 1110, 1055, 740, 694 $cm^{-1}$;
$^1$H-NMR (80 MHz, $CDCl_3$) $\delta$ (TMS): 8.47 (broad s, 1H, pyr), 8.26 (broad s, 1H, pyr), 7.77 (t, J= 2.0Hz, 1H, pyr), 7.26 (m, 5H, Ar), 4.4-4.0 (m, 4H), 3.8-3.5 (m, 4H).

REFERENCE EXAMPLE 8

Phenyl 2,2,3,3,3-pentafluoropropylcarbonate

To a solution of 2,2,3,3,3-pentafluoropropanol (10 g, 0.066 mol) in $CHCl_3$ (250 mL) was added pyridine (10.5 mL, 0.133 mol) and the resulting mixture was cooled to 0 °C. Phenyl chloroformate (12.5 g, 0.08 mol) was added and the reaction mixture was stirred at 0 °C for 1 h. Pyridine was distilled off, more $CHCl_3$ was

added and the resulting solution was washed several times with $NaHCO_3$. The organic layer was dried and the solvent was removed to yield 19.47 g of the desired product as an oil.
IR (KBr) ν: 3062, 2969, 1773, 1587, 1490, 1263, 1202, 1156, 1085 $cm^{-1}$;
$^1$H-NMR (80 MHz, $CDCl_3$) δ (TMS): 7.27 (m, 5H, Ar), 4.66 (t, $J_{HF}$ = 12.6Hz, 2H, $CH_2$).

REFERENCE EXAMPLE 9

N-*tert*-butoxycarbonyl-N-phenyl-N'-(2,2,3,3,3-pentafluoropropoxycarbonyl)-ethylendiamine

To a solution of N-*tert*-butoxycarbonyl-N-phenylethylendiamine (2 g, 8.5 mmol) (obtained according to EP 301751) in 1,1,1-trichloroethane (60 mL) was added 2.8 g of the compound obtained in reference example 8 (2.8 g, 10.2 mmol) and the resulting mixture was heated at reflux for 3 h. The solvent was removed and the residue was diluted with $CHCl_3$ and washed first with $NaHCO_3$ solution and then with brine. The organic phase was dried and the solvent was removed to give 3.92 g of the title compound of the example as an oil.
IR (KBr) ν: 3337, 2972, 1774, 1738, 1684, 1490, 1391, 1257, 1201, 1151 $cm^{-1}$;
$^1$H-NMR (80 MHz, $CDCl_3$) δ (TMS): 7.25 (m, 5H, Ar), 5.5 (broad s., 1H, NH), 4.6 (q, 2H, $OCH_2$), 3.80 (m, 2H, $CH_2$), 3.40 (m, 2H, $CH_2$), 1.41 (s, 9H, $CH_3$).

REFERENCE EXAMPLE 10

N-Phenyl-N'-(2,2,3,3,3-pentafluoropropoxycarbonyl)ethylendiamine

Following the procedure described in reference example 2, but starting from the compound obtained in reference example 9, the title compound of this example was obtained as an oil.
IR (KBr) ν: 3386, 3048, 2958, 1713, 1599, 1503, 1256, 1202, 1149, 751 $cm^{-1}$;
$^1$H-NMR (80 MHz, $CDCl_3$) δ (TMS): 7.19 (m, 2H, Ar), 6.67 (m, 3H, Ar), 5.19 (broad s., 1H, NH), 4.54 (double t, $J_{HF}$ = 12.8Hz, J = 0.7Hz, 2H, $OCH_2$), 3.76 (broad s., 1H, NH), 3.35 (m, 4H, $CH_2CH_2$).

REFERENCE EXAMPLE 11

5-Bromo-3-[N-[2-(2,2,3,3,3-pentafluoropropoxy)carbonylamino]ethyl-N-phenyl]carbamoylpyridine

Following the procedure described in reference example 3, but starting from the compound obtained in reference example 10, the title compound of this example was obtained.
IR (KBr) ν: 3326, 3053, 2954, 1731, 1641, 1590, 1489, 1386, 1295, 1277, 1254, 1203, 1147, 1102, 1019, 699 $cm^{-1}$;
$^1$H-NMR (80 MHz, $CDCl_3$) δ (TMS): 8.51 (d, J = 2.1Hz, 1H, pyr), 8.29 (d, J = 1.5Hz, 1H, pyr), 7.80 (m, 1H, pyr) 7.4-7.0 (m, 5H, Ar), 5.62 (broad s., 1H, NH), 4.52 (double t, $J_{HF}$ = 12.8Hz, J = 0.8Hz, 2H, $OCH_2$), 4.11 (m, 2H, $CH_2$), 3.50 (broad q, J = 5.8Hz, 2H, $CH_2$).

REFERENCE EXAMPLE 12

Phenyl 4-fluorobenzylcarbonate

Following the procedure described in reference example 8, but using 1-fluoro-4-hydroxymethylbenzene instead of 2,2,3,3,3-pentafluoropropanol, the title compound of this example was obtained.
IR (KBr) ν: 3066, 2955, 1742, 1596, 1507, 1490, 1384, 1280, 1222, 1210, 1180, 1157, 847, 756, 706 $cm^{-1}$;
$^1$H-NMR (80 MHz, $CDCl_3$) δ (TMS): 7.5-6.9 (m, 9H, Ar), 5.22 (s, 2H, $CH_2$).

REFERENCE EXAMPLE 13

N-(*tert*-butoxycarbonyl)-N-phenyl-N'-(4-fluorobenzyloxycarbonyl)ethylendiamine

Following the procedure described in reference example 9, but using the compound obtained in reference example 12 instead of the compound obtained in reference example 8, the title compound of this example was obtained.
IR (KBr) ν: 3339, 3057, 2970, 1754, 1691, 1504, 1490, 1388, 1364, 1223, 1155, 824, 759, 698 $cm^{-1}$;

$^1$H-NMR (80 MHz, $CDCl_3$) δ (TMS): 7.5-6.9 (m, 9H, Ar), 5.03 (s, 2H, $CH_2O$), 3.77 (t, J = 6Hz, 2H, $CH_2$), 3.38 (t, J = 6Hz, 2H, $CH_2$), 1.39 (s, 9H, $CH_3$).

REFERENCE EXAMPLE 14

N-Phenyl-N'-(4-fluorobenzyloxycarbonyl)ethylendiamine

Following the procedure described in reference example 2, but starting from the compound obtained in reference example 13, the title compound of this example was obtained as an oil.
IR (KBr) ν: 3382, 3048, 2930, 1680, 1598, 1528, 1506, 1254, 1223, 819, 749, 694 $cm^{-1}$;
$^1$H-NMR (80 MHz, $CDCl_3$) δ (TMS): 7.5-6.5 (m, 9H, Ar), 5.08 (broad s., 3H, $CH_2O + NH$), 3.34 (m, 5H, $CH_2CH_2 + NH$).

REFERENCE EXAMPLE 15

5-Bromo-3-[N-[2-(4-fluorobenzyloxy)carbonylamino]ethyl-N-phenyl]carbamoylpyridine

Following the procedure described in reference example 3, but starting from the compound obtained in reference example 14, the title compound of this example was obtained.
IR (KBr) ν: 3326, 3053, 2934, 1711, 1641, 1589, 1504, 1487, 1385, 1294, 1250, 1221, 1019, 756, 699 $cm^{-1}$;
$^1$H-NMR (80 MHz, $CDCl_3$) δ (TMS): 8.50 (broad s, 1H, pyr), 8.29 (broad s, 1H, pyr), 7.78 (m, 1H, pyr) 7.4-6.9 (m, 9H, Ar), 5.29 (broad s., 1H, NH), 5.05 (s, 2H, $CH_2O$), 4.09 (t, J = 6.3Hz, 2H, $CH_2$), 3.48 (m, 2H, $CH_2$).

REFERENCE EXAMPLE 16

N-(*tert*-butoxycarbonyl)-N-phenyl-N'-[4-(trifluoromethyl)phenylcarbonyl]ethylendiamine

To a solution of N-(*tert*-butoxycarbonyl)-N-phenylethylendiamine (1 g, 4.2 mmol) (obtained according to EP 301751) in anhydrous dichloromethane (30 mL) was added triethylamine (0.708 mL, 5.1 mmol) and the mixture was cooled to 0 °C. *p*-Trifluoromethylbenzoic acid chloride (0.971 g, 4.7 mmol) was added and the reaction mixture was stirred for 2 h. The mixture was diluted with $CHCl_3$, and washed first with $NaHCO_3$, then with brine. The organic phase was dried and the solvent was removed to yield 1.64 g of a yellowish oil.
$^1$H-NMR (80 MHz, $CDCl_3$) δ (TMS): 7.93 (d, J = 8.2Hz, 2H, Ar), 7.66 (d, J = 8.2Hz, 2H, Ar), 7.26 (m, 5H, Ar), 4.3-3.5 (complex signal, 5H, $CH_2CH_2 + NH$), 1.36 (s, 9H, $CH_3$).

REFERENCE EXAMPLE 17

N-Phenyl-N'-[4-(trifluoromethyl)phenylcarbonyl]ethylendiamine

Following the procedure described in reference example 2, but starting from the compound obtained in reference example 16, the title compound of the example was obtained as a solid.
mp: 127-133 °C;
IR (KBr) ν: 3358, 3256, 3022, 2927, 1631, 1600, 1529, 1329, 1162, 1124, 1068, 856, 750, 694 $cm^{-1}$;
$^1$H-NMR (80 MHz, $CDCl_3$) δ (TMS): 7.84 (d, J = 8.5Hz, 2H, Ar), 7.65 (d, J = 8.5Hz, 2H, Ar), 7.19 (m, 2H, Ar), 6.66 (m, 3H, Ar), 3.69 (m, 2H, $CH_2$), 3.46 (m, 2H, $CH_2$).

REFERENCE EXAMPLE 18

5-Bromo-3-[N-[2-[(4-trifluoromethyl)phenyl]carbonylamino]ethyl-N-phenyl]carbamoylpyridine

Following the procedure described in reference example 3, but starting from the compound obtained in reference example 17, the title compound of the example was obtained as a white solid.
mp: 152-153 °C;
IR (KBr) ν: 3375, 3330, 3051, 2946, 1658, 1621, 1538, 1328, 1292, 1153, 1124, 1065, 858, 698 $cm^{-1}$;
$^1$H-NMR (80 MHz, $CDCl_3$) δ (TMS): 8.51 (d, J = 2.1Hz, 1H, pyr), 8.31 (d, J = 1.6Hz, 1H, pyr), 7.96 (d, J = 8.4Hz, 2H, Ar), 7.72 (m, 3H, Ar), 7.4-7.0 (m, 5H, Ar), 4.26 (m, 2H, $CH_2$), 3.78 (m, 2H, $CH_2$).

REFERENCE EXAMPLE 19

Methyl *p*-(2-quinolylmethoxy)phenylacetate

To a mixture of 2-(chloromethyl)quinoline hydrochloride (1g, 4.6mmol) and methyl *p*-hydroxyphenylacetate (0.83 g, 5 mmol) in anhydrous dimethylformamide (20 mL) was added potassium carbonate (2.33 g) and the reaction mixture was heated at 60 °C for 8 h. Dimethylformamide was evaporated and the residue was partitioned between water and chloroform. The organic phase was dried over sodium sulfate and the solvent was removed, to afford 1.68 g of a residue that was purified by chromatography on silica gel (ethyl acetate-hexane 1:1). 1.2 g of the desired product was obtained (yield: 84%).
IR (film) $\nu$: 3024, 2945, 1730, 1595, 1503, 1423, 1242, 1217, 1158, 1050, 826 $cm^{-1}$;
$^1$H-NMR (80 MHz, $CDCl_3$) $\delta$ (TMS): 8.18 (s, 1H, Ar), 8.04 (s, 1H, Ar), 7.8-7.4 (m, 4H, Ar), 7.2-6.9 (m, 4H, Ar), 5.34 (s, 2H, $CH_2O$), 3.64 (s, 3H, $CH_3$), 3.53 (s, 2H, $CH_2CO$).

REFERENCE EXAMPLE 20

*p*-(2-Quinolylmethoxy)phenylacetic acid

To a solution of the product obtained in reference example 19 (1.2 g, 3.9 mmol) in methanol (25 mL) was added potassium carbonate (1.23 g) dissolved in water (12.5 mL) and the reaction mixture was heated at reflux for 1 h. Methanol was evaporated, more water was added and the mixture was extracted with diethyl ether. The aqueous phase was cooled in an ice bath and then was acidified with 5N HCl. The solid formed was filtered and dried to give 0.7 g of the title compound of this example (yield: 61%).
IR (film) $\nu$: 3200-2400, 1699, 1594, 1499, 1286, 1243, 1223, 1139, 1071, 827, 802 $cm^{-1}$;
$^1$H-NMR (80 MHz, $CDCl_3$ + $CD_3OD$) $\delta$ (TMS): 8.3-6.9 (complex signal, 10H, Ar), 5.36 (s, 2H, $CH_2O$), 3.54 (s, 2H, $CH_2CO$).

REFERENCE EXAMPLE 21

Methyl *p*-(2-quinolylmethoxy)benzoate

Following the procedure described in reference example 19, but using methyl *p*-hydroxybenzoate instead of methyl *p*-hydroxyphenylacetate, a crude product was obtained, which was directly used in the next step without further purification.
$^1$H-NMR (80 MHz, $CDCl_3$) $\delta$ (TMS): 8.3-6.9 (complex signal, 10H, Ar), 5.43 (s, 2H, $CH_2O$), 3.87(s, 3N, $CH_3$).

REFERENCE EXAMPLE 22

*p*-(2-Quinolylmethoxy)benzoic acid

Following the procedure described in reference example 20, but starting from the compound obtained in reference example 21, the title compound of the example was obtained (yield: 47%).
mp: >300 °C;
IR (film) $\nu$: 3439, 3058, 1705, 1599, 1502, 1377, 1249, 1171, 1106, 1037, 775 $cm^{-1}$;
$^1$H-NMR (80 MHz, DMSO-$d_6$) $\delta$ (TMS): 8.40 (d, J = 8.4Hz, 1H, Ar), 8.1-7.6 (m, 7H, Ar), 7.05 (m, 2H, Ar), 5.43 (s, 2H, $CH_2O$).

REFERENCE EXAMPLE 23

Methyl 3-hydroxybenzoate

To a solution of 3-hydroxybenzoic acid (1.8 g, 13.2 mmol) in methanol (45 mL) was added concentrated $H_2SO_4$ (0.82 mL) and the mixture was heated at reflux for 18 h. The solvent was removed, water was added and the mixture was extracted with ethyl acetate. The organic layer was dried over sodium sulfate and the solvent was removed to afford 1.98 g of the desired compound (quantitative yield).
IR (film) $\nu$: 3600-3200, 2947, 1694, 1585, 1447, 1433, 1296, 1230, 1103, 997, 755 $cm^{-1}$;
$^1$H-NMR (80 MHz, $CDCl_3$) $\delta$ (TMS): 7.7-7.0 (m, 7H, Ar), 7.05 (complex signal, 4H, Ar), 6.14 (broad s., OH), 3.91(s, 3H, $CH_3$).

REFERENCE EXAMPLE 24

Methyl 3-(2-quinolylmethoxy)benzoate

Following the procedure described in reference example 19, but using the compound obtained in reference example 23 instead of methyl *p*-hydroxyphenylacetate, the title compound of this example was obtained.
IR (film) ν: 3054, 2944, 1713, 1669, 1593, 1580, 1440, 1287, 1216, 1089 $cm^{-1}$;
$^1$H-NMR (80 MHz, $CDCl_3$) δ (TMS): 8.3-7.2 (complex signal, 10H, Ar), 5.41 (s, 2H, $CH_2O$), 3.89(s, 3H, $CH_3$).

REFERENCE EXAMPLE 25

3-(2-Quinolylmethoxy)benzoic acid

Following the procedure described in reference example 20, but starting from the compound obtained in reference example 24, the title compound of this example was obtained (yield: 49%).
mp: 178-182 °C;
IR (film) ν: 3200-2300, 1699, 1591, 1485, 1320, 1285, 1268, 1234, 1218, 1202, 1073, 831, 770, 752 $cm^{-1}$;
$^1$H-NMR (80 MHz, $CDCl_3$ + $CD_3OD$) δ (TMS): 8.4-7.2 (complex signal, 10H, Ar), 5.41(s, 2H, $CH_2O$).

REFERENCE EXAMPLE 26

N-(*tert*-butoxycarbonyl)-N-phenyl-N'-[*p*-(2-quinolylmethoxy)phenylacetyl]ethylendiamine

To a solution of the compound obtained in reference example 20 (1.6 g, 5.5 mmol) in dimethylformamide (50 mL) was added N-(*tert*-butoxycarbonyl)-N-phenylethylendiamine (1.3 g, 5.5 mmol) (obtained according to EP 301751), dicyclohexylcarbodiimide (1.2 g, 5.5 mmol) and 1-hydroxybenzotriazole (0.75 g, 5.5 mmol), and the resulting solution was stirred at room temperature overnight. Dimethylformamide was distilled off and the residue was diluted with chloroform. The organic solution was washed with saturated $NaHCO_3$ solution and then with brine. The solvent was removed, the resulting residue was stirred with ethyl acetate and the white solid formed was filtered. The organic solution was then dried over sodium sulfate and the solvent was removed to afford 4 g of a residue that was purified by chromatography on silica gel (hexane-ethyl acetate mixtures of increasing polarity). 1.60 g of the title compound of the example was obtained (yield: 57%).
IR (KBr) ν: 3315, 3031, 2921, 2844, 1690, 1619, 1565, 1527, 1307, 1240, 1086, 891 $cm^{-1}$.
$^1$H-NMR (80 MHz, $CDCl_3$) δ (TMS): 8.3-8.0 (m, 2H, Ar), 7.9-7.4 (m, 4H, Ar), 7.3-6.9 (m, 9H, Ar), 5.38 (s, 2H, $CH_2O$), 3.8-3.6 (m, 2H, $CH_2$), 3.6-3.2 (m, 2H, $CH_2$), 3.42 (s, 2H, $CH_2CO$), 1.36 (s, 9H, $CH_3$).

REFERENCE EXAMPLE 27

N-phenyl-N'-[*p*-(2-quinolylmethoxy)phenylacetyl]ethylendiamine

Following the procedure described in reference example 2, but starting from the compound obtained in reference example 26, the title compound of this example was obtained as a yellowish solid.
mp: 114-115 °C;
IR (KBr) ν: 3385, 3286, 3043, 2925, 2844, 1636, 1598, 1528, 1503, 1421, 1238, 1175, 1059, 822, 743 $cm^{-1}$;
$^1$H-NMR (80 MHz, $CDCl_3$) δ (TMS): 8.3-8.0 (m, 2H, Ar), 7.9-7.5 (m, 4H, Ar), 7.2-6.9 (m, 6H, Ar), 6.8–6.4 (m, 3H, Ar), 5.37 (s, 2H, $CH_2O$), 3.49 (s, 2H, $CH_2CO$), 3.6-3.1 (m, 4H, $CH_2CH_2$).

REFERENCE EXAMPLE 28

5-Bromo-3-[N-[2-[*p*-(2-quinolylmethoxy)phenyl]acetylamino]ethyl-N-phenyl]carbamoylpyridine

Following the procedure described in reference example 3, but starting from the compound obtained in reference example 27, the title compound of this example was obtained (yield: 92%).
mp: 45-49 °C;
IR (KBr) ν: 3700-3200, 3052, 2925, 1640, 1589, 1502, 1486, 1294, 1239, 825, 746 $cm^{-1}$;
$^1$H-NMR (80 MHz, $CDCl_3$) δ (TMS): 8.47 (d, J = 2.2Hz, 1H, pyr), 8.3-8.0 (m, 3H, Ar), 7.9-7.5 (m, 5H, Ar), 7.22

(d, J = 6Hz, 2H, Ar), 7.3-7.0 (m, 2H, Ar), 7.08 (d, J = 6Hz, 2H, Ar), 7.0-6.8 (m, 3H, Ar), 5.39 (s, 2H, $CH_2O$), 4.2-3.9 (m, 2H, $CH_2$), 3.6-3.3 (m, 2H, $CH_2$), 3.46(s, 2H, $CH_2CO$).

REFERENCE EXAMPLE 29

N-(*tert*-butoxycarbonyl)-N-phenyl-N'-[3-(2-quinolylmethoxy)phenylcarbonyl]ethylendiamine

Following the procedure described in reference example 26, but using the compound obtained in reference example 25 instead of the compound obtained in reference example 20, the title compound of the example was obtained (yield: 79%).
$^1$H-NMR (80 MHz, $CDCl_3$) $\delta$ (TMS): 8.3-8.0 (m, 2H, Ar), 7.9-7.0 (complex signal, 13H, Ar), 5.41 (s, 2H, $CH_2O$), 4.0-3.8 (m, 2H, $CH_2$), 3.7-3.4 (m, 2H, $CH_2$), 1.36 (s, 9H,$CH_3$).

REFERENCE EXAMPLE 30

N-phenyl-N'-[3-(2-quinolylmethoxy)phenylcarbonyl]ethylendiamine

Following the procedure described in reference example 2, but starting from the compound obtained in reference example 29, the title compound of the example was obtained as a yellowish solid (quantitative yield).
mp: 142-145 °C;
IR (KBr) $\nu$: 3370, 3012, 2929, 1625, 1591, 1581, 1517, 1318, 1243, 1141, 1050, 818, 755, 740, 686 $cm^{-1}$;
$^1$H-NMR (80 MHz, $CDCl_3$) $\delta$ (TMS): 8.3-8.0 (m, 2H, Ar), 7.9-7.5 (m, Ar), 7.4-7.0 (m, Ar), 6.9-6.4 (m, Ar), 5.39 (s, 2H, $CH_2O$), 3.65 (t, J = 5.4Hz, 2H, $CH_2$), 3.5-3.2 (m, 2H, $CH_2$), 3.05 (br s., NH).

REFERENCE EXAMPLE 31

5-Bromo-3-[N-[2-[3-(2-quinolylmethoxy)phenyl]carbonylamino]ethyl-N-phenyl]carbamoylpyridine

Following the procedure described in reference example 3, but starting from the compound obtained in reference example 30, the title compound of the example was obtained as a white solid (yield: 57%).
mp: 51-56 °C;
IR (KBr) $\nu$: 3700-3100, 3053, 2931, 1631, 1574, 1528, 1486, 1292, 746, 698 $cm^{-1}$;
$^1$H-NMR (80 MHz, $CDCl_3$) $\delta$ (TMS): 8.52 (d, J = 2Hz, 1H, pyr), 8.30 (d, J = 1.7Hz, 1H, pyr), 8.3-8.0 (m, 2H, Ar), 7.9-7.5 (m, Ar), 7.5-7.0 (m, 6H, Ar), 5.45 (s, 2H, $CH_2O$), 4.4-4.1 (m, 2H, $CH_2$), 3.9-3.6 (m, 2H, $CH_2$).

REFERENCE EXAMPLE 32

N-(*tert*-butoxycarbonyl)-N-phenyl-N'-[*p*-(2-quinolylmethoxy)phenylcarbonyl]ethylendiamine

Following the procedure described in reference example 26, but using the compound obtained in reference example 22 instead of the compound obtained in reference example 20, the title compound of the example was obtained (yield: 62%). $^1$H-NMR (80 MHz, $CDCl_3$) $\delta$ (TMS): 8.3-8.0 (m, 2H, Ar), 7.9-7.5 (m, Ar), 7.4-6.9 (m, Ar), 5.42 (s, 2H, $CH_2O$), 4.0-3.8 (m, 2H, $CH_2$), 3.7-3.4 (m, 4H, $CH_2CH_2$), 1.39 (s, 9H,$CH_3$).

REFERENCE EXAMPLE 33

N-phenyl-N'-[*p*-(2-quinolylmethoxy)phenylcarbonyl]ethylendiamine

Following the procedure described in reference example 2, but starting from the compound obtained in reference example 32, the title compound of the example was obtained as a white solid (quantitative yield).
mp: 189-190 °C;
IR (KBr) $\nu$: 3326, 3035, 2927, 1622, 1599, 1529, 1496, 1419, 1258, 1180, 1058, 843, 819, 746, 693 $cm^{-1}$;
$^1$H-NMR (80 MHz, $CDCl_3$) $\delta$ (TMS): 8.3-8.0 (m, 2H, Ar), 7.9-7.5 (m, Ar), 7.4-7.0 (m, Ar), 6.9-6.5 (m, 3H, Ar), 5.41 (s, 2H, $CH_2O$), 3.65 (t, J = 5.4Hz, 2H, $CH_2$), 3.5-3.2 (m, 2H, $CH_2$).

REFERENCE EXAMPLE 34

5-Bromo-3-[N-[2-[*p*-(2-quinolylmethoxy)phenyl]carbonylamino]ethyl-N-phenyl]carbamoylpyridine

Following the procedure described in reference example 3, but starting from the compound obtained in reference example 33, the title compound of the example was obtained as a white solid (yield: 82%).
mp: 160-161 °C;
IR (KBr) ν: 3365, 2937, 1636, 1600, 1591, 1495, 1290, 1280, 1245, 1179, 1056, 745, 698 $cm^{-1}$;
$^1$H-NMR (80 MHz, $CDCl_3$) δ (TMS): 8.47 (broad s, 1H, pyr), 8.3-8.0 (m, 3H, Ar), 7.9-7.5 (m, Ar), 7.4-6.9 (m, Ar), 5.44 (s, 2H, $CH_2O$), 4.4-4.1 (m, 2H, $CH_2$), 3.8-3.5 (m, 2H, $CH_2$).

EXAMPLE 1

5-Bromo-3-[N-(2-ethoxycarbonylamino)ethyl-N-phenyl]carbamoyl-1-propylpyridinium iodide

To a solution of the compound obtained in reference example 3 (0.84 g, 2.14 mmol) in acetonitrile (5 mL), was added propyl iodide (16 mL) and the mixture was heated at reflux for 48 h. The resulting solution was concentrated *in vacuo* , the residue was dissolved in dichloromethane and precipitated with ether and acetone. The solid was filtered and dried to yield 1.09 g of the title compound of this example (yield: 93%).
mp: 86-93 °C;
IR (KBr) ν: 3600-3200, 2969, 1698, 1648, 1512, 1487, 1397, 1280, 1248, 1034, 702, 673 $cm^{-1}$;
$^1$H-NMR (80 MHz, $CDCl_3$) δ (TMS): 9.42 (s, 1H, pyr), 9.36 (s, 1H, pyr), 8.41 (s, 1H, pyr), 7.36 (m, 5H, Ar), 5.99 (broad s., 1H, NH), 4.78 (broad t, J = 7.1Hz, 2H, $CH_2$-pyr), 4.06 (q, J = 7Hz, 2H, $OCH_2$), 4.02 (m, 2H, $CH_2$), 3.47 (m, 2H, $CH_2$), 1.84 (m, 2H, pyr-$CH_2\underline{CH_2}$), 1.20 (t, J = 7Hz, 3H, $OCH_2\underline{CH_3}$), 0.76 (t, J = 7.4Hz, 3H, pyr-$(CH_2)_2\underline{CH_3}$).

EXAMPLE 2

5-Bromo-3-[N-(2-ethoxycarbonylamino)ethyl-N-phenyl]carbamoyl-1-propylpyridinium chloride

A solution of the compound obtained in example 1 (550 mg, mmol) in 70% aqueous methanol was eluted through an ionic interchange column IRA410 (chloride form). The filtrate was concentrated to dryness and recrystallized from ether-acetone to give the title compound of the example (395 mg, yield: 83%).
mp: 72-80 °C;
IR (KBr) ν: 3700-3100, 2966, 1694, 1648, 1520, 1487, 1395, 1280, 1251, 1037, 744, 702, 675 $cm^{-1}$;
$^1$H-NMR (80 MHz, $CDCl_3$) δ (TMS): 9.77 (broad s, 2H, pyr), 8.42 (s, 1H, pyr), 7.27 (m, 5H, Ar), 4.83 (t, J = 6.9Hz, 2H, $CH_2$-pyr), 4.06 (q, J = 7.1Hz, 2H, $OCH_2$), 4.01 (m, 2H, $CH_2$), 3.55 (broad s., 2H), 1.79 (m, 2H, pyr-$CH_2\underline{CH_2}$), 1.19 (t, J = 7.1Hz, 3H, $OCH_2\underline{CH_3}$), 0.70 (t, J = 7.3Hz, 3H, pyr-$(CH_2)_2\underline{CH_3}$).

EXAMPLE 3

5-Bromo-3-[N-[2-(2-iodoethoxy)carbonylamino]ethyl-N-phenyl]carbamoyl-1-propylpyridinium iodide

Following the procedure described in example 1, but starting from the compound obtained in reference example 6, the title compound of the example was obtained.
mp: 62-76 °C;
IR (KBr) ν: 3600-3100, 2959, 1702, 1646, 1511, 1487, 1396, 1280, 1244, 1139, 741, 701, 673 $cm^{-1}$;
$^1$H-NMR (80 MHz, $CDCl_3$) δ (TMS): 9.40 (s, 2H, pyr), 8.43 (s, 1H, pyr), 7.38 (m, 5H, Ar), 6.29 (broad s., 1H, NH), 4.78 (t, J = 7.1Hz, 2H, $CH_2$-pyr), 4.4-4.0 (m, 4H), 3.57 (m, 2H, $CH_2$), 3.25 (t, J = 6.9Hz, 2H, $CH_2$), 1.80 (m, 2H, pyr-$CH_2\underline{CH_2}$), 0.76 (t, J = 7.1Hz, 3H, pyr-$(CH_2)_2\underline{CH_3}$).

EXAMPLE 4

5-Bromo-3-[N-[2-(2-iodoethoxy)carbonylamino]ethyl-N-phenyl]carbamoyl-1-propylpyridinium chloride

Following the procedure described in example 2, but starting from the compound obtained in example 3, the title compound of this example was obtained.
mp: 73-82 °C;

IR (KBr) ν: 3600-3100, 2958, 1702, 1648, 1518, 1487, 1398, 1280, 1248, 745, 702, 675 $cm^{-1}$;
$^1$H-NMR (80 MHz, $CDCl_3$) δ (TMS): 9.86 (s, 1H, pyr), 9.49 (s, 1H, pyr), 8.43 (s, 1H, pyr), 7.34 (m, 5H, Ar), 4.80 (t, J= 7.1Hz, 2H, $CH_2$-pyr), 4.27 (m, 2H, $CH_2$), 4.03 (m, 2H, $CH_2$), 3.60 (m, 2H, $CH_2$), 3.25 (m, 2H, $CH_2$), 1.75 (m, 2H, pyr-$CH_2\underline{CH_2}$), 0.70 (t, J= 7Hz, 3H, pyr-$(CH_2)_2\underline{CH_3}$).

EXAMPLE 5

5-Bromo-3-[N-[2-(2-oxo-1,3-oxazolidin-3-yl)ethyl-N-phenyl]carbamoyl]-1-propylpyridinium iodide

Following the procedure described in example 1, but starting from the compound obtained in reference example 7, the title compound of this example was obtained.
mp: 62-73 °C;
IR (KBr) ν: 3600-3200 ($H_2O$), 2963, 2923, 1728, 1648, 1487, 1426, 1395, 1266, 1178, 1107, 756, 701, 674 $cm^{-1}$;
$^1$H-NMR (80 MHz, $CDCl_3$) δ (TMS): 9.60 (s, 1H, pyr), 9.17 (s, 1H, pyr), 8.41 (s, 1H, pyr), 7.6-7.2 (m, 5H, Ar), 4.83 (t, J= 7.3Hz, 2H, $CH_2$-pyr), 4.4–4.0 (m, 4H), 3.9-3.5 (m, 4H), 1.78 (m, 2H, pyr-$CH_2\underline{CH_2}$), 0.77 (t, J= 7.4Hz, 3H, pyr-$(CH_2)_2CH_3$).

EXAMPLE 6

5-Bromo-3-[N-[2-(2-oxo-1,3-oxazolidin-3-yl)ethyl-N-phenyl]carbamoyl]-1-propylpyridinium chloride

Following the procedure described in example 2, but starting from the compound obtained in example 5, the title compound of the example was obtained.
mp: 33-40 °C;
IR (KBr) ν: 3700-3200 ($H_2O$), 2959, 1728, 1648, 1487,1427, 1399, 1268, 1179, 1108, 1051, 760, 703, 675 $cm^{-1}$;
$^1$H-NMR (80 MHz, $CDCl_3$) δ (TMS): 9.94 (s, 1H, pyr), 9.37 (s, 1H, pyr), 8.35 (s, 1H, pyr), 7.7-7.2 (m, 5H, Ar), 4.95 (t, J= 6.9Hz, 2H, $CH_2$-pyr), 4.5-4.0 (m, 4H), 3.9-3.5 (m, 4H), 1.84 (m, 2H, pyr-$CH_2\underline{CH_2}$), 0.77 (t, J= 7.2Hz, 3H, pyr-$(CH_2)_2\underline{CH_3}$).

EXAMPLE 7

5-Bromo-3-[N-[2-(2-iodoethyl)aminocarbonyloxylethyl-N-phenyl]carbamoyl-1-propylpyridinium iodide

The title compound of the example was obtained from N-*tert*-butoxycarbonyl-N-phenylethanolamine (obtained according to EP 301751) by a procedure similar to the one described above, which comprises the following steps: reaction with phenyl chloroformate; reaction of the carbonate thus obtained with 2-iodoethylamine; deprotection of the *tert*-butoxycarbonyl group to give the free amine; reaction of the resulting amine with 5-bromonicotinic acid chloride; and finally, alkylation of the pyridinic N with propyl iodide. All reactions were carried out under experimental conditions similar to those described above.
mp: 71-82 °C;
IR (KBr) ν: 3600-3100, 2959, 1704, 1648, 1513, 1486, 1395, 1243, 1171, 702, 675 $cm^{-1}$;
$^1$H-NMR (80 MHz, $CDCl_3$) δ (TMS): 9.47 (broad s, 1H, pyr), 9.20 (broad s, 1H, pyr), 8.29 (s, 1H, pyr), 7.40 (m, 5H, Ar), 6.56 (broad s., 1H, NH), 4.90 (t, J= 7.0Hz, 2H, $CH_2$-pyr), 4.22 (m, 4H), 3.6-3.2 (m, 4H), 1.85 (m, 2H, pyr-$CH_2\underline{CH_2}$), 0.84 (t, J= 7.1Hz, 3H, pyr-$(CH_2)_2\underline{CH_3}$).

EXAMPLE 8

5-Bromo-3-[N-[2-(2,2,3,3,3-pentafluoropropoxy)carbonylamino]ethyl-N-phenyl]carbamoyl-1-propylpyridinium iodide

Following the procedure described in example 1, but starting from the ompound obtained in reference example 11, the title compound of the example was obtained.
IR (KBr) ν: 3600-3100, 2958, 1702, 1648, 1504, 1487, 1394, 1245, 1219, 755 $cm^{-1}$;
$^1$H-NMR (80 MHz, $CDCl_3$) δ (TMS): 9.41 (s, 1H, pyr), 9.32 (s, 1H, pyr), 8.40 (s, 1H, pyr), 7.31 (m, 5H, Ar), 6.56 (broad t, J= 5.7Hz, 1H, NH), 4.75 (t, J= 7.1Hz, 2H, $CH_2$-pyr), 4.25 (t, $J_{HF}$= 13.1Hz, 2H, $OCH_2$), 4.04 (m, 2H, $CH_2$), 3.59 (m, 2H, $CH_2$), 1.78 (m, 2H, pyr-$CH_2\underline{CH_2}$), 0.75 (t, J= 7.3Hz, 3H, pyr-$(CH_2)_2\underline{CH_3}$).

EXAMPLE 9

5-Bromo-3-[N-[2-(4-fluorobenzyloxy)carbonylamino]ethyl-N-phenyl]carbamoyl-1-propylpyridinium iodide

Following the procedure described in example 1, but starting from the compound obtained in reference example 15, the title compound of the example was obtained.
IR (KBr) $\nu$: 3600-3200, 2962, 1727, 1648, 1487, 1397, 1280, 1252, 1199, 1144, 1103, 1053 $cm^{-1}$;
$^1$H-NMR (80 MHz, $CDCl_3$) $\delta$ (TMS): 9.39 (s, 1H, pyr), 9.24 (s, 1H, pyr), 8.33 (s, 1H, pyr), 7.5-6.9 (m, 9H, Ar), 6.25 (broad t, J = 5.8Hz, 1H, NH), 5.03 (s, 2H, $OCH_2$), 4.72 (t, J = 7Hz, 2H, $CH_2$-pyr), 4.05 (m, 2H, $CH_2$), 3.57 (m, 2H, $CH_2$), 1.75 (m, 2H, pyr-$CH_2\underline{CH_2}$), 0.73 (t, J = 7.3Hz, 3H, pyr-$(CH_2)_2\underline{CH_3}$).

EXAMPLE 10

5-Bromo-3-[N-[2-[(4-trifluoromethyl)phenyl]carbonylaminolethyl-N-phenyl]carbamoyl-1-propylpyridinium iodide

Following the procedure described in example 1, but starting from the compound obtained in reference example 18, the title compound of the example was obtained.
IR (KBr) $\nu$: 3600-3100, 2962, 1648, 1529, 1487, 1398, 1324, 1164, 1123, 1066, 858, 751 $cm^{-1}$;
$^1$H-NMR (80 MHz, $CDCl_3$) $\delta$ (TMS): 9.68 (s, 1H, pyr), 8.93 (s, 1H, pyr), 8.37 (s, 1H, pyr), 8.18 (d, J = 8.2Hz, 2H, Ar), 7.66 (d, J = 8.2Hz, 2H, Ar), 7.51 (m, 2H, Ar), 7.26 (m, 3H, Ar), 4.68 (t, J = 7.1Hz, 2H, $CH_2$-pyr), 4.16 (m, 2H, $CH_2$), 3.88 (m, 2H, $CH_2$), 1.80 (m, 2H, pyr-$CH_2\underline{CH_2}$), 0.75 (t, J = 7.3Hz, 3H, pyr-$(CH_2)_2\underline{CH_3}$).

EXAMPLE 11

5-Bromo-3-[N-[2-[(4-trifluoromethyl)phenyl]carbonylaminolethyl-N-phenyl]carbamoyl-1-propylpyridinium chloride

Following the procedure described in example 2, but starting from the compound obtained in example 10, the title compound of the example was obtained.
mp: 112-119 °C;
IR (KBr) $\nu$: 3600-3100, 3034, 2962, 1648, 1538, 1487, 1401, 1324, 1164, 1123, 1067 $cm^{-1}$;
$^1$H-NMR (80 MHz, $CDCl_3$) $\delta$ (TMS): 10.35 (s, 1H, pyr), 9.59 (broad s, 1H, pyr), 8.81 (s, 1H, pyr), 8.33 (d, J = 8.6Hz, 2H, Ar), 7.65 (d, J = 8.6Hz, 2H, Ar), 7.51 (m, 2H, Ar), 7.22 (m, 3H, Ar), 4.68 (t, J = 6.8Hz, 2H, $CH_2$-pyr), 4.12 (m, 2H, $CH_2$), 3.90 (m, 2H, $CH_2$), 1.80 (m, 2H, pyr-$CH_2\underline{CH_2}$), 0.72 (t, J = 7.4Hz, 3H, pyr-$(CH_2)_2\underline{CH_3}$).

EXAMPLE 12

5-Bromo-3-[N-[2-[*p*-(2-quinolylmethoxy)phenyl]acetylaminolethyl-N-phenyl]carbamoylpyridinium iodide

Following the procedure described in example 1, but starting from the compound obtained in reference example 28, a yellow residue was obtained, which was purified by chromatography on silica gel (chloroform-methanol, 5:1 and then 3:1) to give the title compound of the example.
mp: 89-93 °C;
IR (KBr) $\nu$: 3600-3100, 3034, 2923, 1648, 1502, 1487, 1423, 1392, 1295, 1238, 1222, 826, 745, 701 $cm^{-1}$;
$^1$H-NMR (80 MHz, $CDCl_3$) $\delta$ (TMS): 9.49 (s, 1H, pyr), 9.11(s, 1H, pyr), 8.31 (s, 1H, pyr), 8.3-8.0 (m, 2H, Ar), 7.9-7.5 (m, Ar), 7.5-7.1 (m, Ar), 7.0-6.8 (m, Ar), 5.33 (s, 2H, $CH_2O$), 4.70 (broad t, 2H, $CH_2$-pyr), 4.00 (m, 2H, $CH_2$), 3.59 (broad s, 4H, $CH_2CO$ + $CH_2$), 1.74 (m, 2H, pyr-$CH_2\underline{CH_2}$), 0.71 (t, J = 7.1Hz, 3H, pyr-$(CH_2)_2\underline{CH_3}$).

EXAMPLE 13

5-Bromo-3-[N-[2-[*p*-(2-quinolylmethoxy)phenyl]acetylamino]ethyl-N-phenyl]carbamoylpyridinium chloride

Following the procedure described in example 2, but starting from the compound obtained in example 12, the title compound of the example was obtained.
mp: 124-127 °C;

IR (KBr) ν: 3600-3100, 3043, 3013, 2961,1657, 1502, 1486, 1423, 1392, 1343, 1303, 1239, 1222, 820, 744, 697 $cm^{-1}$;
$^{1}$H-NMR (80 MHz, $CDCl_3$) δ (TMS): 10.18 (s, 1H, pyr), 9.13 (s, 1H, pyr), 8.34 (s, 1H, pyr), 8.3-8.1 (m, 2H, Ar), 8.0-7.5 (m, Ar), 7.5-7.1 (m, Ar), 7.0-6.7 (m, Ar), 5.35 (s, 2H, $CH_2O$), 4.78 (m, 2H, $CH_2$-pyr), 3.90 (m, 2H, $CH_2$), 3.67(broad s, 4H, $CH_2CO$ + $CH_2$), 2.0-1.5 (m, 2H, pyr-$CH_2\underline{CH_2}$), 0.69 (t, J=6.9Hz, 3H, pyr-$(CH_2)_2\underline{CH_3}$).

EXAMPLE 14

5-Bromo-3-[N-[2-[3-(2-quinolylmethoxy)phenyl]carbonylamino]ethyl-N-phenyl]carbamoylpyridinium iodide

Following the procedure described in example 1, but starting from the compound obtained in reference example 31, and then purifying the residue obtained by chromatography on silica gel (chloroform-methanol, 5:1 and then 3:1), the title compound of the example was obtained as a yellow solid (yield: 94%).
mp: 103-112 °C;
IR (KBr) ν: 3600-3100, 2957, 1648, 1574, 1520, 1477, 1421, 1391, 1294, 1219, 750 $cm^{-1}$;
$^{1}$H-NMR (80 MHz, $CDCl_3$) δ (TMS): 9.47 (s, 1H, pyr), 9.06 (br s, 1H, pyr), 8.35 (s, 1H, pyr), 8.3-7.9 (m, 2H, Ar), 7.9-7.0 (complex signal, 13H, Ar), 5.42 (s, 2H, $CH_2O$), 4.66 (broad t, J= 7.lHz, 2H, $CH_2$-pyr), 4.13 (m, 2H, $CH_2$), 3.83 (m, 2H, $CH_2$), 1.9-1.6 (m, 2H, pyr-$CH_2\underline{CH_2}$), 0.69 (t, J= 7.4Hz, 3H, pyr-$(CH_2)_2\underline{CH_3}$).

EXAMPLE 15

5-Bromo-3-[N-[2-[3-(2-quinolylmethoxy)phenyl]carbonylamino]ethyl-N-phenyl]carbamoylpyridinium chloride

Following the procedure described in example 2, but starting from the compound obtained in example 14, the title compound of the example was obtained (yield: 94%).
mp: 100-108 °C;
IR (KBr) ν: 3600-3100, 2960, 1648, 1580, 1529, 1486, 1421, 1395, 1298, 1220, 750 $cm^{-1}$;
$^{1}$H-NMR (80 MHz, $CDCl_3$) δ (TMS): 10.04 (s, 1H, pyr), 9.13 (s, 1H, pyr), 8.39 (s, 1H, pyr), 8.3-7.9 (m, 2H, Ar), 7.9-7.7 (m, Ar), 7.7-7.3 (m, Ar), 7.3-7.0 (m, Ar), 5.43 (s, 2H, $CH_2O$), 4.69 (broad t, J= 6.9Hz, 2H, $CH_2$-pyr), 4.08 (m, 2H, $CH_2$), 3.86 (m, 2H, $CH_2$), 2.0-1.6 (m, 2H, pyr-$CH_2\underline{CH_2}$), 0.65 (t, J= 7.3Hz, 3H, pyr-$(CH_2)_2\underline{CH_3}$).

EXAMPLE 16

5-Bromo-3-[N-[2-[*p*-(2-quinolylmethoxy)phenyl]carbonylamino]ethyl-N-phenyl]carbamoylpyridinium iodide

Following the procedure described in example 1, but starting from the compound obtained in reference example 34, and then purifying the residue obtained by chromatography on silica gel (chloroform-methanol, 5:1 and then 3:1), the title compound of the example was obtained as a yellow solid (yield: 92%).
mp: 110-115 °C;
IR (KBr) ν: 3700-3100, 2959, 1641, 1598, 1491, 1393, 1292, 1244, 1175, 751 $cm^{-1}$;
$^{1}$H-NMR (80 MHz, $CDCl_3$) δ (TMS): 9.47 (s, 1H, pyr), 9.07 (s, 1H, pyr), 8.35 (s, 1H, pyr), 8.3-6.9 (complex signal, 15H, Ar), 5.38 (s, 2H, $CH_2O$), 4.67 (broad t, J= 7.1Hz, 2H, $CH_2$-pyr), 4.11 (m, 2H, $CH_2$), 3.82 (m, 2H, $CH_2$), 2.0-1.5 (m, 2H, pyr-$CH_2\underline{CH_2}$), 0.72 (t, J= 7Hz, 3H, pyr-$(CH_2)_2\underline{CH_3}$).

EXAMPLE 17

5-Bromo-3-[N-[2-[*p*-(2-quinolylmethoxy)phenyl]carbonylamino]ethyl-N-phenyl]carbamoylpyridinium chloride

Following the procedure described in example 2, but starting from the compound obtained in example 16, the title compound of the example was obtained (yield: 69%).
mp: 116-122 °C;
IR (KBr) ν: 3600-3100, 2959, 1648, 1598, 1493, 1395, 1293, 1244, 1176, 751 $cm^{-1}$;
$^{1}$H-NMR (80 MHz, $CDCl_3$) δ (TMS): 9.91(s, 1H, pyr), 9.30 (s, 1H, pyr), 8.40 (s, 1H, pyr), 8.3-7.9 (m, Ar), 7.9-7.3 (m, Ar), 7.3-6.9 (m, Ar), 5.37 (s, 2H, $CH_2O$), 4.70 (broad t, 2H, $CH_2$-pyr), 4.05 (m, 2H, $CH_2$), 3.83 (m, 2H, $CH_2$), 1.9-1.5 (m, 2H, pyr-$CH_2\underline{CH_2}$), 0.65 (t, J= 7.3Hz, 3H, pyr-$(CH_2)_2\underline{CH_3}$).

## Claims

1. A compound of formula **I**:

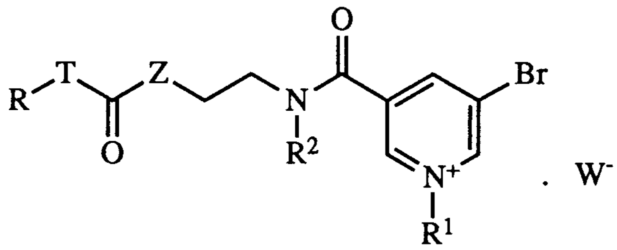

I

wherein:

$R^1$ represents a $C_{1-6}$ alkyl or aryl-$C_{1-6}$ alkyl group;

$R^2$ represents aryl;

Z is -O- or -$NR^3$-;

T is -O-, -$NR^4$- or a single bond;

R represents $C_{1-18}$ alkyl, $C_{1-18}$ haloalkyl, aryl, heteroaryl, aryl-$C_{1-6}$ alkyl or heteroaryl-$C_{1-6}$ alkyl, and in addition, when T represents a single bond, R can also represent a phenyl or phenylmethyl radical substituted in the para or meta position by a 2-quinolylmethoxy group;

$R^3$ and $R^4$ independently represent hydrogen, $C_{1-4}$ alkyl, aryl or $C_{1-4}$ alkylcarbonyl; in addition, when Z = $NR^3$ and T = $NR^4$, $R^3$ and $R^4$ may form a ring together with the group -N-C(=O)-N- to which they are bonded of formula

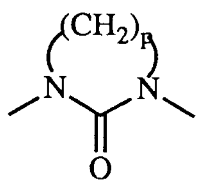

wherein p represents 2, 3 or 4, or when Z = $NR^3$, $R^3$ may be bonded to R to form a ring together with the group -T-C(=O)-N- to which $R^3$ and R are bonded of formula

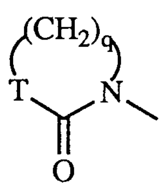

wherein q is 2, 3 or 4;

aryl in the above meanings is a phenyl group which can be optionally substituted by one or more groups chosen from halogen, trifluoromethyl, $C_{1-4}$ alkyl, $C_{1-4}$ alkoxy or hydroxy;

heteroaryl in the above meanings is any radical from an aromatic heterocycle selected from pyridine, pyrimidine, pyrazine, pyridasine, pyrrole, imidazole, triazole, furan, thiophene, thiazole, oxazole and isoxazole, and that may be optionally substituted by a group chosen from halogen, $C_{1-4}$ alkyl, $C_{1-4}$ alkoxy or hydroxy; and

$W^-$ is a counter anion;

and the solvates thereof.

2. A compound according to claim 1 wherein $W^-$ is a pharmaceutically acceptable anion.

**3.** A compound according to claims 1 or 2 wherein
$R^1$ represents $C_{1-3}$ alkyl; and
$R^2$, R, T, Z and $W^-$ have the previously defined meaning.

**4.** A compound according to claims 1, 2 or 3 wherein
$R^1$ represents $C_{1-3}$ alkyl;
$R^2$ represents Ph; and
R, T, Z and $W^-$ have the previously defined meaning.

**5.** A compound according to claims 1, 2, 3 or 4 wherein
$R^1$ represents $C_{1-3}$ alkyl;
$R^2$ represents Ph;
R represents $C_{1-18}$ alkyl, $C_{1-18}$ haloalkyl, aryl, heteroaryl, aryl-$C_{1-6}$ alkyl or heteroaryl-$C_{1-6}$ alkyl;
Z is -$NR^3$- and T represents -O- or a single bond, or Z is -O- and T represents -$NR^4$-; and
$W^-$, $R^3$ and $R^4$ have the previously defined meaning.

**6.** A compound according to claims 1, 2, 3 or 4 wherein
$R^1$ represents $C_{1-3}$ alkyl;
$R^2$ represents Ph;
R represents a phenyl or phenylmethyl radical substituted in the para or meta position by a 2-quinolylmethoxy group;
T represents a single bond;
Z is -$NR^3$-; and
$W^-$ and $R^3$ have the previously defined meaning.

**7.** 5-Bromo-3-[N-(2-ethoxycarbonylamino)ethyl-N-phenyl]carbamoyl-1-propylpyridinium iodide or any other pharmaceutically acceptable salt or solvate thereof.

**8.** 5-Bromo-3-[N-[2-(2-iodoethyl)aminocarbonyloxy]ethyl-N-phenyl]carbamoyl-1-propylpyridinium iodide or any other pharmaceutically acceptable salt or solvate thereof.

**9.** 5-Bromo-3-[N-[2-[(4-trifluoromethyl)phenyl]carbonylamino]ethyl-N-phenyl]-carbamoyl-1-propylpyridinium chloride or any other pharmaceutically acceptable salt or solvate thereof.

**10.** 5-Bromo-3-[N-[2-[*p*-(2-quinolylmethoxy)phenyl]acetylamino]ethyl-N-phenyl]carbamoylpyridinium chloride or any other pharmaceutically acceptable salt or solvate thereof.

**11.** A process for preparing the compounds of formula **I** as defined in claim 1 which comprises reacting a compound of general formula **II**,

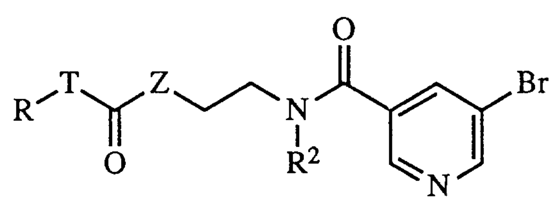

**(II)**

wherein R, $R^2$, Z and T have the previously defined meaning, with a compound of general formula $R^1$-Y (**III**, wherein $R^1$ has the previously defined meaning and Y represents a good leaving group, such as a halogen atom, methylsulfonate, *p*-toluenesulfonate) in a manner being known per se, and optionally changing the nature of the counter anion by means of ion-exchange chromatography or selective salt precipitation.

**12.** A pharmaceutical composition which comprises an effective amount of at least one compound of formula **I** as defined in any one of claims 1 to 10 in admixture with one or more pharmaceutically

acceptable excipients.

**13.** The use of at least one compound of formula **I** as defined in any one of claims 1 to 10 for the manufacture of a medicament for the treatment or prevention of the diseases in which PAF is involved.

**14.** The use according to claim 13 for the manufacture of a medicament for the treatment of inflammation.

**15.** A compound of the formula

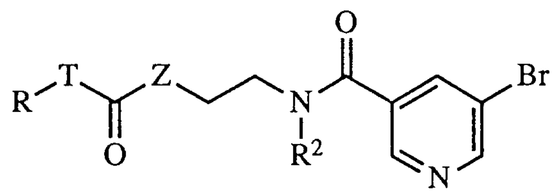

wherein R, $R^2$, Z and T are as defined in claim 1.

European Patent Office

# EUROPEAN SEARCH REPORT

Application Number
EP 94 10 2138

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| A | EP-A-0 382 380 (TAKEDA CHEMICAL INDUSTRIES) 16 August 1990<br>* claim 1 *<br>--- | 1-15 | C07D213/82<br>A61K31/44<br>C07D413/12<br>C07D401/12<br>//(C07D401/12, 213:00, 215:00), (C07D413/12, 213:00,263:00) |
| A,D | EP-A-0 301 751 (TAKEDA CHEMICAL INDUSTRIES) 1 February 1989<br>* claim 1 *<br>--- | 1-15 | |
| P,A | EP-A-0 530 444 (AMERICAN CYANAMID COMPANY) 10 March 1993<br>* claim 1 *<br>----- | 1-15 | |
| | | | TECHNICAL FIELDS SEARCHED (Int.Cl.5) |
| | | | C07D<br>A61K |

The present search report has been drawn up for all claims

| Place of search | Date of completion of the search | Examiner |
|---|---|---|
| MUNICH | 20 April 1994 | Gettins, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document